(19) Europäisches Patentamt
European Patent Office
Office européen des brevets

(11) Veröffentlichungsnummer: **0 627 427 A1**

# (12) EUROPÄISCHE PATENTANMELDUNG

(21) Anmeldenummer: **94105773.9**

(22) Anmeldetag: **14.04.94**

(51) Int. Cl.5: **C07D 401/04**, A61K 31/445, C07D 211/90, C07D 401/14, C07D 215/14, C07D 215/12

(30) Priorität: **27.04.93 DE 4313692**

(43) Veröffentlichungstag der Anmeldung: **07.12.94 Patentblatt 94/49**

(84) Benannte Vertragsstaaten: **AT BE CH DE DK ES FR GB GR IE IT LI LU MC NL PT SE**

(71) Anmelder: **BAYER AG**

**D-51368 Leverkusen (DE)**

(72) Erfinder: **Stoltefuss, Jürgen, Dipl.-Ing.**
**Parkstrasse 20**
**D-42781 Haan (DE)**
Erfinder: **Goldmann, Siegfried, Dr.**
**Am Osterholz 91**
**D-42327 Wuppertal (DE)**
Erfinder: **Straub, Alexander, Dr.**
**Moospfad 30**
**D-42113 Wuppertal (DE)**
Erfinder: **Bechem, Martin, Dr.**
**Hans-Böckler-Strasse 102**
**D-42111 Wuppertal (DE)**
Erfinder: **Gross, Rainer, Prof. Dr.**
**Platzhofstrasse 23**
**D-42115 Wuppertal (DE)**
Erfinder: **Hebisch, Siegbert, Dr.**
**Johann-Breuker-Platz 8**
**D-46244 Bottrop (DE)**
Erfinder: **Hütter, Joachim, Dr.**
**Teschen-Sudberger-Strasse 13**
**D-42349 Wuppertal (DE)**
Erfinder: **Rounding, Howard-Paul**
**Pahlkestrasse 15**
**D-42115 Wuppertal (DE)**

## (54) 3-Chinolyl substituierte Dihydropyridine, Verfahren zu ihrer Herstellung und ihre Verwendung in Arzneimitteln.

(57) Die Erfindung betrifft neue 3-Chinolyl substituierte Dihydropyridine der allgemeinen Formel (I)

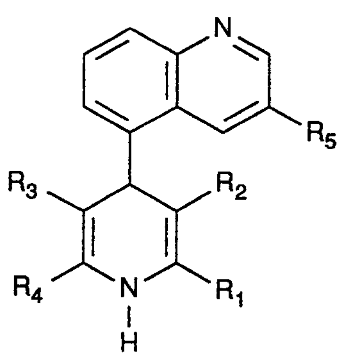

(I),

in welcher $R_1$ bis $R_5$ die in der Beschreibung angegebene Bedeutung haben, Verfahren zu ihrer Herstellung und ihre Verwendung in Arzneimitteln, insbesondere in Mitteln zur Behandlung von Herz-Kreislauferkrankungen.

EP 0 627 427 A1

Die Erfindung betrifft neue 3-Chinolyl substituierte Dihydropyridine, Verfahren zu ihrer Herstellung und ihre Verwendung in Arzneimitteln, insbesondere in Mitteln zur Behandlung von Herz-Kreislauferkrankungen.

Es ist bereits bekannt, daß 1,4-Dihydropyridine gefäßerweiternde Eigenschaften besitzen und als Coronarmittel und Antihypertensiva verwendet werden können. Weiterhin ist bekannt daß 1,4-Dihydropyridine eine Hemmung der Kontraktionskraft von glatten und cardialen Muskeln bewirken und zur Behandlung von Coronar-und Gefäßerkrankungen eingesetzt werden können.

Ferner sind aus der US 5 100 900 schon 4-Chinolyl-dihydropyridine mit positiv inotroper Wirkung bekannt.

Die vorliegende Erfindung betrifft neue 3-Chinolyl substituierte Dihydropyridine der allgemeinen Formel (I)

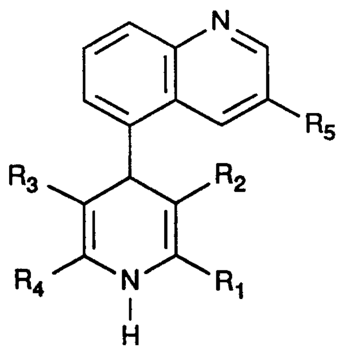

(I)

in welcher

| | |
|---|---|
| $R^1$ und $R^4$ | gleich oder verschieden sind und für Wasserstoff, Amino, Cyano, Formyl, Trifluormethyl oder für geradkettiges oder verzweigtes Alkyl mit bis zu 8 Kohlenstoffatomen stehen, das gegebenenfalls durch Hydroxy oder durch eine Gruppe der Formel $-NR^6R^7$, $-O-CO-R^8$, $-O-(CH_2)_a-OR^{8'}$ oder $-O-(CH_2)_b-NR^9R^{10}$ substituiert ist, worin |
| $R^6$, $R^7$, $R^9$ und $R^{10}$ | gleich oder verschieden sind und Wasserstoff, Phenyl oder geradkettiges oder verzweigtes Alkyl mit bis zu 6 Kohlenstoffatomen bedeuten, |
| $R^8$ und $R^{8'}$ | gleich oder verschieden sind und geradkettiges oder verzweigtes Alkyl mit bis zu 6 Kohlenstoffatomen bedeuten, und |
| a und b | gleich oder verschieden sind und eine Zahl 2, 3, 4 oder 5 bedeuten, |
| $R^2$ | für eine Gruppe der Formel $-CO-NR^{11}R^{12}$ oder $-CO-A-R^{13}$ steht, worin |
| $R^{11}$ und $R^{12}$ | gleich oder verschieden sind und Wasserstoff, einen geradkettigen, verzweigten oder cyclischen, gesättigten oder ungesättigten Kohlenwasserstoffrest mit bis zu 8 Kohlenstoffatomen bedeuten, der gegebenenfalls durch Halogen, Hydroxy, Cyano oder durch Aryl, Aryloxy, Arylthio mit jeweils 6 bis 10 Kohlenstoffatomen oder durch einen 5- bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder |
| $R^{11}$ und $R^{12}$ | gemeinsam unter Einbezug des Stickstoffatoms einen 3-bis 8-gliedrigen, gesättigen oder ungesättigten Heterocyclus bilden, der gegebenenfalls durch ein Sauerstoffatom oder durch einen Rest der Formel $S(O)_d$, -CO- oder $-NR^{15}$ unterbrochen |

sein kann,

worin

d eine Zahl 0, 1 oder 2 bedeutet,

$R^{15}$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio Alkoxycarbonyl mit jeweils bis zu 8 Kohlenstoffatomen, Halogenalkyl, Halogenalkoxy oder Halogenalkylthio mit jeweils bis zu 4 Kohlenstoffatomen substituiert ist, oder

einen cyclischen, gesättigten oder ungesättigten, geradkettigen oder verzweigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5-bis 7-gliedrigen, gesättigen oder ungesättigten Heterocyclus mit bis zu 3 Heteroatomen aus der Reihe S, N oder O substituiert ist, wobei die Cyclen ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkoxy oder Alkylthio mit jeweils bis zu 4 Kohlenstoffatomen, Halogen, Aryl mit 6 bis 10 Kohlenstoffatomen, einen 5- bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das seinerseits durch Aryl mit 6 bis 10 Kohlenstoffatomen substituiert sein kann,

A eine direkte Bindung oder ein Sauerstoffatom bedeutet,

$R^{13}$ Wasserstoff oder Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeutet, wobei die Cyclen gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder

einen cyclischen, geradkettigen oder verzweigten, gesättigten oder ungesättigten Kohlenwasserstoffrest mit bis zu 10 Kohlenstoffatomen bedeutet, der gegebenenfalls bis zu 3-fach gleich oder verschieden durch Sauerstoff oder durch - CO-, -CO-NH-, -O-CO-, -CO-O-, -NH-CO-, $-SO_2$-NH-, -NH-$SO_2$-, -$S(O)_e$-oder -$N^{16}$- unterbrochen ist,

worin

e die oben angegebene Bedeutung von d hat und mit dieser gleich oder verschieden ist,

$R^{16}$ die oben angegebene Bedeutung von $R^{15}$ hat und mit dieser gleich oder verschieden ist,

oder der Kohlenwasserstoffrest gegebenenfalls bis zu 3-fach gleich oder verschieden durch Aryliden mit 6 bis 10 Kohlenstoffatomen oder heterocyclische Reste der Formeln

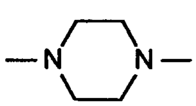 , 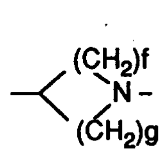 , 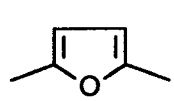 oder 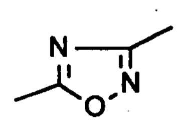

unterbrochen ist,

worin

f und g gleich oder verschieden sind und eine Zahl 1 oder 2 bedeuten,

und wobei Aryliden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein kann,

und der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, - $O-NO_2$, geradkettiges oder verzweigtes Alkylthio, Alkoxy oder Acyloxy mit jeweils bis zu 8 Kohlenstoffatomen oder durch Aryl, Aryloxy oder Arylthio mit jeweils 6 bis 10 Kohlenstoffatomen oder durch einen 5-bis 7-gliedrigen, gesättigten oder ungesättigen Heterocyclus mit bis zu 3 Heteroatomen aus der Reihe S, N oder O substituiert ist, wobei die Cyclen ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder

der Kohlenwasserstoffrest gegebenenfalls durch eine Gruppe der Formel $-CO_2-R^{17}$, $-CONR^{18}R^{19}$, - $NR^{20}R^{21}$, $NH-SO_2-X$ und/oder $-NR^{22}-CO_2R^{23}$ substituiert ist,

worin

$R^{17}$ die oben angegebene Bedeutung von $R^{15}$ hat und mit dieser gleich oder verschieden ist

und

$R^{18}$, $R^{19}$, $R^{20}$, $R^{21}$, $R^{22}$ und $R^{23}$ die oben angegebene Bedeutung von $R^{11}$ und $R^{12}$ haben und mit diesen gleich oder verschieden sind,

und

X Phenyl bedeutet, das gegebenenfalls durch Methyl substituiert ist,

$R^3$ für Cyano, Nitro oder Formyl steht,

oder

$R^3$ und $R^4$ gemeinsam einen Rest der Formel

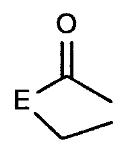

bilden,

worin

E ein Sauerstoff-oder Schwefelatom oder die - $CH_2$-Gruppe bedeutet,

$R^5$ für einen cyclischen, geradkettigen oder verzweigten, gesättigten oder ungesättigten Kohlenwasserstoffrest mit bis zu 12 Kohlenstoffatomen steht, der gegebenenfalls bis zu 2-fach gleich oder verschieden durch Sauerstoff oder Schwefel unterbrochen ist,

und der gegebenenfalls bis zu 3-fach gleich oder verschieden durch Cycloalkyl mit 3 bis 8 Kohlenstoffatomen, geradkettiges oder verzweigtes Acyloxy mit bis zu 4 Kohlenstoffatomen, Halogen, Nitro, Cyano, Hydroxy oder durch Aryl, Aryloxy oder Arylthio mit jeweils 6 bis 10 Kohlenstoffatomen oder durch einen 5-bis 7-gliedrigen, gesättigten oder ungesättigen gegebenenfalls kondensierten Heterocyclus mit bis zu 5 Heteroatomen aus der Reihe S, N oder O substituiert ist, wobei die Cyclen ihrerseits bis zu 3-fach gleich oder verschieden durch Halogen, Cyano, Nitro, Hydroxy oder durch geradkettiges oder verzweigtes Alkyl oder Alkoxy mit jeweils bis zu 4 Kohlenstoffatomen, Trifluormethyl, Trifluormethoxy, Trifluormethylthio oder durch eine Gruppe der For-

mel - $NR^{24}R^{25}$ substituiert sein können,
worin

$R^{24}$ und $R^{25}$ die oben angegebene Bedeutung von $R^{11}$ und $R^{12}$ haben und mit dieser gleich oder verschieden sind,

oder der Kohlenwasserstoffrest gegebenenfalls durch eine Gruppe der Formel $-CO_2-R^{26}$, $CONR^{27}R^{28}$, $-NR^{29}R^{30}$, $-NR^{31}-CO_2R^{32}$ oder $-NR^{33}-SO_2R^{34}$ substituiert ist,
worin

$R^{26}$ die oben angegebene Bedeutung von $R^{15}$ hat und mit dieser gleich oder verschieden ist
und

$R^{27}$, $R^{28}$, $R^{29}$, $R^{30}$, $R^{31}$, $R^{32}$, $R^{33}$ und $R^{34}$ die oben angegebene Bedeutung von $R^{11}$ und $R^{12}$ haben und mit diesen gleich oder verschieden sind,
oder

$R^5$ für einen 5-bis 7-gliedrigen, gesättigten oder ungesättigten Heterocyclus mit bis zu 4 Heteroatomen aus der Reihe S, N oder O steht, der gegebenenfalls bis zu 3-fach gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono- oder -Dialkylamino substituiert ist,
oder

$R^5$ für eine Gruppe der Formel $D-R^{35}$ steht,
worin

D die CO- oder $-S(O)_h$-Gruppe oder ein Sauerstoffatom bedeutet,
worin

h eine Zahl 0, 1 oder 2 bedeutet,
und

$R^{35}$ Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeutet, die gegebenenfalls bis zu 3-fach gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono- oder - Dialkylamino substituiert sind,
oder

$R^{35}$ Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel unterbrochen ist,
und der gegebenenfalls durch Halogen, Aryl, Aryloxy oder Arylthio mit jeweils 6 bis 10 Kohlenstoffatomen oder durch einen 5-bis 7-gliedrigen, gesättigten oder ungesättigen Heterocyclus mit bis zu 3 Heteroatomen aus der Reihe S, N oder O substituiert ist, wobei die Cyclen ihrerseits durch Halogen, Trifluormethyl, Methyl, Methoxy, Nitro oder Methylthio substituiert sein können, oder
durch eine Gruppe der Formel $-NR^{36}R^{37}$ substituiert ist
worin

$R^{36}$ und $R^{37}$ die oben angegebene Bedeutung von $R^{11}$ und $R^{12}$ haben und mit dieser gleich oder verschieden sind,

und deren Salze,
mit der Maßgabe, daß $R^5$ nicht für gegebenenfalls halogensubstituiertes Pyridyl oder Thienyl stehen darf.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Bevorzugt sind Verbindungen der allgemeinen Formel (I),
in welcher

| | |
|---|---|
| $R^1$ und $R^4$ | gleich oder verschieden sind und für Wasserstoff, Amino, Cyano, Formyl, Trifluormethyl oder für geradkettiges oder verzweigtes Alkyl mit bis zu 4 Kohlenstoffatomen stehen, das gegebenenfalls durch Hydroxy, durch eine Gruppe der Formel $-NR^6R^7$, $-O-CO-R^8$, $-O-(CH_2)_a-OR^{8'}$ oder $-O-(CH_2)_{b'}-NR^9R^{10}$ substituiert ist, worin |
| $R^6$, $R^7$, $R^9$ und $R^{10}$ | gleich oder verschieden sind und Wasserstoff oder geradkettiges oder verzweigtes Alkyl mit bis zu 4 Kohlenstoffatomen bedeuten, |
| $R^8$ und $R^{8'}$ | gleich oder verschieden sind und geradkettiges oder verzweigtes Alkyl mit bis zu 4 Kohlenstoffatomen bedeuten, und |
| a und b | gleich oder verschieden sind und eine Zahl 2, 3, 4 oder 5 bedeuten, |
| $R^2$ | für eine Gruppe der Formel $-CO-NR^{11}R^{12}$ oder $-CO-A-R^{13}$ steht, worin |
| $R^{11}$ und $R^{12}$ | gleich oder verschieden sind und Wasserstoff, einen geradkettigen, verzweigten oder cyclischen, gesättigten oder ungesättigten Kohlenwasserstoffrest mit bis zu 6 Kohlenstoffatomen bedeuten, der gegebenenfalls durch Fluor, Chlor, Hydroxy, Phenyl oder Pyridyl substituiert ist, wobei die Cyclen ihrerseits durch Fluor, Chlor oder durch Alkyl, Alkoxy, Alkylthio oder Alkoxycarbonyl mit jeweils bis zu 2 Kohlenstoffatomen, Trifluormethyl oder Trifluormethoxy substituiert sein können, oder<br>Phenyl oder Pyridyl bedeuten, die gegebenenfalls durch Fluor, Chlor oder durch Alkyl, Alkoxy, Alkylthio oder Alkoxycarbonyl mit jeweils bis zu 2 Kohlenstoffatomen, Trifluormethyl oder Trifluormethoxy substituiert sind, oder |
| $R^{11}$ und $R^{12}$ | gemeinsam unter Einbezug des Stickstoffatoms einen 3-bis 8-gliedrigen, gesättigen oder ungesättigten Heterocyclus bilden, der gegebenenfalls durch ein Sauerstoffatom oder durch einen Rest der Formel $S(O)_d$, -CO- oder $-NR^{15}$ unterbrochen sein kann, worin |
| d | eine Zahl 0, 1 oder 2 bedeutet, |
| $R^{15}$ | Wasserstoff oder Phenyl bedeutet, das gegebenenfalls durch Fluor, Chlor, Methyl, Ethyl, Methoxy, Ethoxy, Trifluormethyl oder Trifluormethoxy substituiert ist, oder<br>einen cyclischen, gesättigten oder ungesättigten, geradkettigen oder verzweigten Kohlenwasserstoffrest mit bis zu 4 Kohlenstoffatomen bedeutet, der gegebenenfalls durch Fluor, Chlor, Phenyl oder durch einen 5-bis 7-gliedrigen, gesättigten oder ungesättigten Heterocyclus mit bis zu 3 Heteroatomen aus der Reihe S, N oder O substituiert ist, wobei die Cyclen ihrerseits durch Fluor, Chlor, Methyl, Methoxy, Methylthio, Trifluormethyl oder Trifluormethoxy substituiert sein können, |
| A | eine direkte Bindung oder ein Sauerstoffatom bedeutet, |
| $R^{13}$ | Wasserstoff, Phenyl oder Pyridyl bedeutet, die gegebenenfalls durch Fluor, Chlor, Methyl, Methoxy, Methylthio, Trifluormethyl oder Trifluormethoxy substituiert sind, oder<br>einen cyclischen, geradkettigen oder verzweigten, gesättigten oder ungesättigten Kohlenwasserstoffrest mit bis zu 8 Kohlenstoffatomen bedeutet, der gegebenen- |

falls bis zu 2-fach gleich oder verschieden durch Sauerstoff oder durch - CO-, -CO-NH-, -O-CO-, -CO-O-, -NH-CO-, $-SO_2-NH-$, $-NH-SO_2-$, $-S(O)_e-$oder $-NR^{16}-$ unterbrochen ist,
worin

e die oben angegebene Bedeutung von d hat und mit dieser gleich oder verschieden ist,

$R^{16}$ die oben angegebene Bedeutung von $R^{15}$ hat und mit dieser gleich oder verschieden ist,

oder der Kohlenwasserstoffrest gegebenenfalls bis zu 2-fach gleich oder verschieden durch Aryliden mit 6 bis 10 Kohlenstoffatomen oder heterocyclische Reste der Formeln

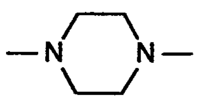 , 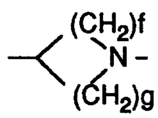 , 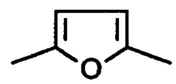 oder 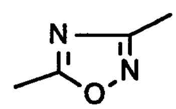

unterbrochen ist,
worin

f und g gleich oder verschieden sind und eine Zahl 1 oder 2 bedeuten,

und der Kohlenwasserstoffrest gegebenenfalls bis zu 2-fach gleich oder verschieden durch Cyclopropyl, Cyclobutyl, Cyclopentyl, Cyclohexyl, Fluor, Chlor, Nitro, Cyano, Hydroxy, $-O-NO_2$, geradkettiges oder verzweigtes Alkylthio, Alkoxy oder Acyloxy mit jeweils bis zu 4 Kohlenstoffatomen oder durch Phenyl, Phenoxy, Phenylthio oder Pyridyl substituiert ist, wobei die Cyclen ihrerseits durch Fluor, Chlor, Cyano, Methyl, Methoxy, Methylthio, Trifluormethyl oder Trifluormethoxy substituiert sein können, oder

der Kohlenwasserstofftest gegebenenfalls durch eine Gruppe der Formel $-CO_2-R^{17}$, $-CONR^{18}R^{19}$, $-NR^{20}R^{21}$, $-NH-SO_2-X$ und/oder $-NR^{22}-CO_2R^{23}$ substituiert ist,
worin

$R^{17}$ die oben angegebene Bedeutung von $R^{15}$ hat und mit dieser gleich oder verschieden ist
und

$R^{18}$, $R^{19}$, $R^{20}$, $R^{21}$, $R^{22}$ und $R^{23}$ die oben angegebene Bedeutung von $R^{11}$ und $R^{12}$ haben und mit diesen gleich oder verschieden sind,
und

X Phenyl bedeutet, das gegebenenfalls durch Methyl substituiert ist,

$R^3$ für Cyano, Nitro oder Formyl steht,

oder
$R^3$ und $R^4$ gemeinsam einen Rest der Formel

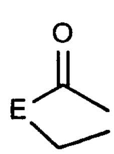

bilden,
worin

E ein Sauerstoff-oder Schwefelatom oder die $-CH_2$-Gruppe bedeutet,

$R^5$ für einen cyclischen, geradkettigen oder verzweigten, gesättigten oder ungesättigten Kohlenwasserstoffrest mit bis zu 10

Kohlenstoffatomen steht, der gegebenenfalls durch Sauerstoff oder Schwefel unterbrochen ist, und der gegebenenfalls bis zu 2-fach gleich oder verschieden durch Cyclopropyl, Cyclobutyl, Cyclopentyl, Cyclohexyl, Fluor, Chlor, Brom, Acyloxy mit bis zu 2 Kohlenstoffatomen, Cyano, Hydroxy oder durch Phenyl, Phenyloxy, Phenylthio oder durch einen 5-bis 7-gliedrigen, gesättigten oder ungesättigen Heterocyclus mit bis zu 3 Heteroatomen aus der Reihe S, N oder O substituiert ist, wobei die Phenyl-und die Hetero-Cyclen ihrerseits bis zu 2-fach gleich oder verschieden durch Fluor, Chlor, Brom, Hydroxy, Methyl, Ethyl, Methoxy, Ethoxy, Trifluormethyl, Trifluormethoxy oder Trifluormethylthio oder durch eine Gruppe der Formel - $NR^{24}R^{25}$ substituiert sein können,

worin

$R^{24}$ und $R^{25}$ die oben angegebene Bedeutung von $R^{11}$ und $R^{12}$ haben und mit dieser gleich oder verschieden sind,

oder der Kohlenwasserstoffrest gegebenenfalls durch eine Gruppe der Formel $-CO_2-R^{26}$, - $CONR^{27}R^{28}$, - $NR^{29}R^{30}$, - $NR^{31}-CO_2R^{32}$ oder - $NR^{33}-SO_2R^{34}$ substituiert ist,

worin

$R^{26}$ die oben angegebene Bedeutung von $R^{15}$ hat und mit dieser gleich oder verschieden ist

und

$R^{27}$, $R^{28}$, $R^{29}$, $R^{30}$, $R^{31}$, $R^{32}$, $R^{33}$ und $R^{34}$ die oben angegebene Bedeutung von $R^{11}$ und $R^{12}$ haben und mit diesen gleich oder verschieden sind,

oder

$R^5$ für einen 5-bis 7-gliedrigen, gesättigten oder ungesättigten Heterocyclus mit bis zu 4 Heteroatomen aus der Reihe S, N oder O steht, der gegebenenfalls bis zu 2-fach gleich oder verschieden durch Fluor, Chlor, Brom, Cyano, Nitro oder durch Alkyl, Alkoxy, Alkylthio, Alkoxycarbonyl, Halogenalkyl, Halogenalkoxy oder Halogenalkylthio mit jeweils bis zu 2 Kohlenstoffatomen, Amino oder durch $C_1$-$C_4$-Mono- oder -Dialkylamino substituiert ist,

oder

$R^5$ für eine Gruppe der Formel $D-R^{35}$ steht,

worin

D die CO- oder - $S(O)_h$-Gruppe oder ein Sauerstoffatom bedeutet,

worin

h eine Zahl 0, 1 oder 2 bedeutet,

und

$R^{35}$ Phenyl oder einen 5-bis 7-gliedrigen, gesättigten oder ungesättigten Heterocyclus mit bis zu 3 Heteroatomen aus der Reihe S, N oder O bedeutet, die gegebenenfalls bis zu 2-fach gleich oder verschieden durch Fluor, Chlor, Brom, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen, Amino oder durch $C_1$-$C_4$-Mono- oder -Dialkylamino substituiert sind,

oder

$R^{35}$ Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel unterbrochen ist, und der gegebenenfalls durch Fluor, Chlor, Phenyl, Phenoxy

oder Phenylthio substituiert sein kann, oder durch eine Gruppe der Formel - $NR^{36}R^{37}$ substituiert ist worin

$R^{36}$ und $R^{37}$ die oben angegebene Bedeutung von $R^{11}$ und $R^{12}$ haben und mit dieser gleich oder verschieden sind,

und deren Salze,
mit der Maßgabe, daß $R^5$ nicht für gegebenenfalls halogensubstituiertes Pyridyl oder Thienyl stehen darf.

Besonders bevorzugt sind Verbindungen der allgemeinen Formel (I),
in welcher

$R^1$ und $R^4$ gleich oder verschieden sind und für Wasserstoff, Amino, Trifluormethyl, Methyl oder Ethyl stehen,

$R^2$ für eine Gruppe der Formel -CO-$NR^{11}R^{12}$ oder -CO-A-$R^{13}$ steht, worin

$R^{11}$ und $R^{12}$ gleich oder verschieden sind und Wasserstoff, einen geradkettigen, verzweigten oder cyclischen, gesättigten oder ungesättigten Kohlenwasserstoffrest mit bis zu 6 Kohlenstoffatomen bedeuten, oder Phenyl bedeuten, das gegebenenfalls durch Fluor, Chlor, Methyl oder Methoxy substituiert ist,

A eine direkte Bindung oder ein Sauerstoffatom bedeutet,

$R^{13}$ Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel oder durch -CO-NH-, -O-CO-, -CO-O-, -NH-CO-, -$SO_2$-NH-, -NH-$SO_2$- oder -$NR^{16}$- unterbrochen ist, worin

$R^{16}$ Wasserstoff, geradkettiges oder verzweigtes Alkyl mit bis zu 4 Kohlenstoffatomen bedeutet,

oder der Kohlenwasserstoffrest durch heterocyclische Reste der Formeln

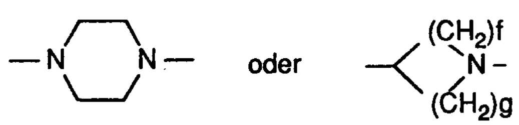

unterbrochen ist, worin

f und g gleich oder verschieden sind und eine Zahl 1 oder 2 bedeuten,

und der Kohlenwasserstoffrest gegebenenfalls durch Cyclopropyl, Cyclobutyl, Cyclopentyl, Cyclohexyl, Fluor, Chlor, Nitro, Cyano, Hydroxy oder durch Phenyl, Phenoxy, Phenylthio oder Pyridyl substituiert ist, die ihrerseits durch Fluor, Chlor, Methyl, Methoxy, Methylthio, Trifluormethyl oder Trifluormethoxy substituiert sein können, oder der Kohlenwasserstoffrest gegebenenfalls durch eine Gruppe der Formel -$CO_2R^{17}$, -$CONR^{18}R^{19}$, -$NR^{20}R^{21}$, - NH-$SO_2$-X und/oder -$NR^{22}$-$CO_2R^{23}$ substituiert ist, worin

$R^{20}$ und $R^{21}$ gleich oder verschieden sind und Wasserstoff, einen geradkettigen, verzweigten oder cyclischen, gesättigten oder ungesättigten Kohlenwasserstoffrest mit bis zu 6 Kohlenstoffatomen bedeuten, das gegebenenfalls durch Fluor, Chlor, Pyridyl oder Phenyl substituiert ist, das seinerseits durch Fluor, Chlor, Methyl oder Methoxy substituiert sein kann, oder Phenyl bedeuten, das gegebenenfalls durch Fluor, Chlor, Methyl oder Methoxy substituiert ist,
oder

$R^{20}$ und $R^{21}$ gemeinsam unter Einbezug des Stickstoffatoms einen 5-bis 6-gliedrigen, gesättigen oder ungesättigten Heterocyclus bilden, der gegebenenfalls bis zu 2 weitere Heteroatome aus der Reihe S, N oder O enthalten kann und

der gegebenenfalls auch geradkettiges oder verzweigtes Alkyl mit bis zu 4 Kohlenstoffatomen, Benzyl oder Phenyl substituiert ist,

X Phenyl bedeutet, das gegebenenfalls durch Methyl substituiert ist,

$R^{17}$, $R^{18}$, $R^{19}$, $R^{22}$ und $R^{23}$ gleich oder verschieden sind und Wasserstoff, geradkettiges oder verzweigtes Alkyl mit bis zu 6 Kohlenstoffatomen bedeuten, das gegebenenfalls durch Phenyl substituiert ist, oder

Phenyl bedeuten, das gegebenenfalls durch Fluor, Chlor oder Brom substituiert ist,

$R^3$ für Cyano, Nitro oder Formyl steht,

oder

$R^3$ und $R^4$ gemeinsam einen Rest der Formel

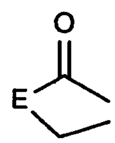

bilden,

worin

E ein Sauerstoff-oder Schwefelatom oder die - $CH_2$-Gruppe bedeutet,

$R^5$ für einen cyclischen, geradkettigen oder verzweigten, gesättigten oder ungesättigten Kohlenwasserstoffrest mit bis zu 8 Kohlenstoffatomen steht, der gegebenenfalls durch Sauerstoff oder Schwefel unterbrochen ist,

und der gegebenenfalls durch Cyclopropyl, Cyclobutyl, Cyclopentyl, Cyclohexyl, Fluor, Chlor, Nitro, Cyano, Hydroxy oder durch Phenyl, Phenyloxy, Phenylthio oder durch einen 5-bis 7-gliedrigen, gesättigten oder ungesättigen Heterocyclus mit bis zu 2 Heteroatomen aus der Reihe S, N oder O substituiert ist, wobei die Phenyl-und die Hetero-Cyclen ihrerseits bis zu 2-fach gleich oder verschieden durch Fluor, Chlor, Methyl, Methoxy, Trifluormethyl oder Trifluormethoxy oder durch eine Gruppe der Formel $-NR^{24}R^{25}$ substituiert sein können,

worin

$R^{24}$ und $R^{25}$ die oben angegebene Bedeutung von $R^{11}$ und $R^{12}$ haben und mit dieser gleich oder verschieden sind,

oder der Kohlenwasserstoffrest gegebenenfalls durch eine Gruppe der Formel $-CO_2-R^{26}$, $-CONR^{27}R^{28}$, $-NR^{29}R^{30}$, $-NR^{31}-CO_2R^{32}$ oder $-NR^{33}-SO_2R^{34}$ substituiert ist,

worin

$R^{26}$ Alkyl mit 1-4 C-Atomen oder Phenyl bedeutet,

und

$R^{27}$, $R^{28}$, $R^{29}$, $R^{30}$, $R^{31}$, $R^{32}$, $R^{33}$ und $R^{34}$ die oben angegebene Bedeutung von $R^{11}$ und $R^{12}$ haben und mit diesen gleich oder verschieden sind,

oder

$R^5$ für einen 5-bis 6-gliedrigen, gesättigten oder ungesättigten Heterocyclus mit bis zu 2 Heteroatomen aus der Reihe S, N oder O steht, der gegebenenfalls durch Fluor, Chlor, Methyl, Methoxy, Methylthio oder Trifluormethyl substituiert ist,

oder

$R^5$ für eine Gruppe der Formel $D-R^{35}$ steht,

worin

D die $-S(O)_h$-Gruppe oder ein Sauerstoffatom bedeutet,

worin

h eine Zahl 0, 1 oder 2 bedeutet,
und
$R^{35}$ Phenyl bedeutet, das gegebenenfalls durch Fluor, Chlor, Brom, Methyl, Methoxy, Methylthio,Trifluormethyl, Amino oder durch $C_1$-$C_2$-Mono- oder - Dialkylamino substituiert ist,
oder
$R^{35}$ Wasserstoff, einen cyclischen, geradkettigen oder verzweigten, gesättigten oder ungesättigten Kohlenwasserstoffrest mit bis zu 6 Kohlenstoffatomen bedeutet, der gegebenenfalls durch Sauerstoff oder Schwefel unterbrochen ist,
und der gegebenenfalls durch Fluor, Chlor oder Phenyl substituiert ist, oder
durch eine Gruppe der Formel - $NR^{36}R^{37}$ substituiert ist
worin
$R^{36}$ und $R^{37}$ die oben angegebene Bedeutung von $R^{11}$ und $R^{12}$ haben und mit dieser gleich oder verschieden sind,

und deren Salze,
mit der Maßgabe, daß $R^5$ nicht für gegebenenfalls halogensubstituiertes Pyridyl oder Thienyl stehen darf.

Ganz besonders bevorzugt sind Verbindungen der allgemeinen Formel (I),
in welcher

$R^1$ für Wasserstoff, Methyl, Ethyl, Trifluormethyl oder Amino steht,
$R^4$ für Wasserstoff, Methyl oder Amino steht,
$R^2$ für eine Gruppe der Formel -CO-$NR^{11}R^{12}$ oder -CO-A-$R^{13}$ steht,
worin
$R^{11}$ und $R^{12}$ gleich oder verschieden sind und Wasserstoff oder einen geradkettigen, verzweigten oder cyclischen, gesättigten oder ungesättigten Kohlenwasserstoffrest mit bis zu 6 Kohlenstoffatomen bedeuten,
A eine direkte Bindung oder ein Sauerstoffatom bedeutet,
$R^{13}$ Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel oder durch -O-CO-, -CO-O- oder $NR^{16}$ unterbrochen ist,
worin
$R^{16}$ Wasserstofff oder Methyl bedeutet

und der Kohlenwasserstoffrest gegebenenfalls durch Cyclopropyl, Cyclobutyl, Cyclopentyl, Cyclohexyl, Fluor, Chlor, Nitro, Cyano, Hydroxy, -O-$NO_2$ oder durch Phenyl, Phenoxy, Phenylthio oder Pyridyl substituiert ist, die ihrerseits durch Fluor, Chlor, Methyl, Methoxy, Methylthio, Trifluormethyl oder Trifluormethoxy substituiert sein kann,
oder

der Kohlenwasserstoffrest gegebenenfalls durch eine Gruppe der Formel -$CO_2$-$R^{17}$, -NH-$SO_2$-X und/oder -$NR^{20}R^{21}$ substituiert ist,
worin
$R^{17}$ Wasserstoff, Methyl oder Ethyl bedeutet,
$R^{20}$ und $R^{21}$ gleich oder verschieden sind und Wasserstoff oder einen geradkettigen, verzweigten oder cyclischen, gesättigten oder ungesättigten Kohlenwasserstoffrest mit bis zu 5 Kohlenstoffatomen bedeuten, der gegebenenfalls durch Phenyl substituiert ist, das seinerseits durch Fluor, Chlor, Methyl oder Methoxy substituiert sein kann,
oder
$R^{20}$ und $R^{21}$ gemeinsam unter Einbezug des Stickstoffatoms einen Piperidin- oder Piperazinring bilden, der gegebenenfalls durch Methyl, Ethyl, Phenyl oder Benzyl substituiert sein kann,
X Phenyl bedeutet, das gegebenenfalls durch Methyl substituiert ist,
$R^3$ für Cyano oder Nitro steht,

oder
$R^3$ und $R^4$ gemeinsam einen Rest der Formel

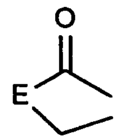

bilden,
worin

E ein Sauerstoff- oder Schwefelatom oder die $-CH_2$-Gruppe bedeutet,

$R^5$ für einen cyclischen, geradkettigen oder verzweigten, gesättigten oder ungesättigten Kohlenwasserstoffrest mit bis zu 6 Kohlenstoffatomen steht, der gegebenenfalls durch Sauerstoff oder Schwefel unterbrochen ist,
und der gegebenenfalls durch Cyclopropyl, Cyclobutyl, Cyclopentyl, Cyclohexyl, Fluor, Chlor, Cyano, Hydroxy oder durch Phenyl, Phenyloxy, Phenylthio oder durch einen 5- bis 7-gliedrigen, gesättigten oder ungesättigten Heterocyclus mit bis zu 2 Heteroatomen aus der Reihe S, N oder O substituiert ist, wobei die Cyclen ihrerseits durch Fluor, Chlor, Methyl, Methoxy, Trifluormethyl oder Trifluormethoxy oder durch eine Gruppe der Formel $-NR^{24}R^{25}$ substituiert sein können,
worin

$R^{24}$ und $R^{25}$ Wasserstoff, Phenyl, Benzyl oder geradkettiges oder verzweigtes Alkyl mit bis zu 4 Kohlenstoffatomen bedeuten,
oder der Kohlenwasserstoffrest gegebenenfalls durch eine Gruppe der Formel $-NR^{29}R^{30}$ substituiert ist,
worin

$R^{29}$ und $R^{30}$ gleich oder verschieden sind und Wasserstoff, Phenyl oder geradkettiges oder verzweigtes Alkyl mit bis zu 4 Kohlenstoffatomen bedeuten, das gegebenenfalls durch Phenyl substituiert ist,
oder

$R^5$ für einen 5- bis 6-gliedrigen, gesättigten oder ungesättigten Heterocyclus mit bis zu 2 Heteroatomen aus der Reihe S, N oder O steht, der gegebenenfalls durch Fluor, Chlor, Methyl, Methoxy, Methylthio oder Trifluormethyl substituiert ist,
oder

$R^5$ für eine Gruppe der Formel $D-R^{35}$ steht,
worin

D ein Sauerstoff- oder Schwefelatom bedeutet,
und

$R^{35}$ Phenyl bedeutet, das gegebenenfalls durch Fluor, Chlor, Methyl, Methoxy, Methylthio, Trifluormethyl, Amino oder durch $C_1$-$C_2$-Mono- oder -Dialkylamino substituiert ist,
oder $R^{35}$ Wasserstoff, einen cyclischen, geradkettigen oder verzweigten, gesättigten oder ungesättigten Kohlenwasserstoffrest mit bis zu 6 Kohlenstoffatomen bedeutet, der gegebenenfalls durch Fluor, Chlor oder Phenyl substituiert ist,

und deren Salze,
mit der Maßgabe, daß $R^5$ nicht für gegebenenfalls halogensubstituiertes Pyridyl oder Thienyl stehen darf.

Außerdem wurde ein Verfahren zur Herstellung der erfindungsgemäßen Verbindungen der allgemeinen Formel (I) gefunden, dadurch gekennzeichnet,
daß man im Fall, daß $R^3$ für Cyano, Nitro oder Formyl steht,

[A] Verbindungen der allgemeinen Formel (II)

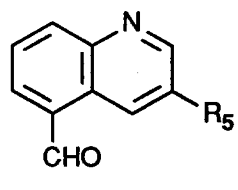

(II)

in welcher
$R^5$ die oben angegebene Bedeutung hat,
zunächst mit Acylverbindungen der allgemeinen Formel (III)

$R^3$-CO-$CH_2$-$R^4$ (III)

in welcher
$R^3$ und $R^4$ die oben angegebene Bedeutung haben
gegebenenfalls unter Isolierung der Ylidenverbindungen der allgemeinen Formel (IV)

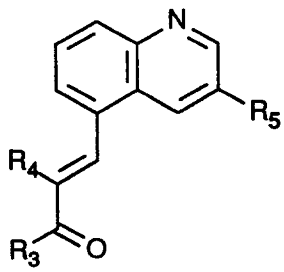
(IV)

in welcher
$R^3$, $R^4$ und $R^5$ die oben angegebene Bedeutung haben
umsetzt, anschließend mit Verbindungen der Formel (V)

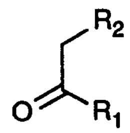
(V)

in welcher
$R^1$ und $R^2$ die oben angegebene Bedeutung haben,
und einer reaktiven Ammoniumverbindung, z.B. Ammoniumacetat, oder direkt mit Enamino-Verbindungen der allgemeinen Formel (VI)

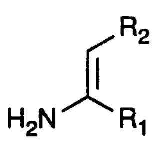
(VI)

in welcher
$R^1$ und $R^2$ die oben angegebene Bedeutung haben,
in inerten Lösemitteln umsetzt,
oder
[B] Verbindungen der allgemeinen Formel (II) zunächst mit Verbindungen der allgemeinen Formel (V), gegebenenfalls unter Isolierung der Ylidenverbindungen der allgemeinen Formel (VII)

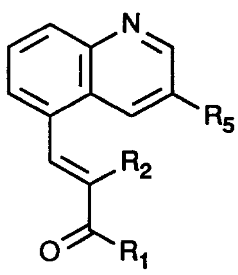

(VII)

in welcher
$R^1$, $R^2$ und $R^5$ die oben angegebene Bedeutung haben,
umsetzt und anschließend entweder mit Verbindungen der allgemeinen Formel (III) in Gegenwart von Ammoniumverbindungen oder direkt mit Verbindungen der allgemeinen Formel (VIII)

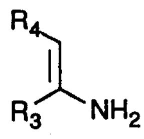

(VIII)

in welcher
$R^3$ und $R^4$ die oben angegebenene Bedeutung haben,
umsetzt
oder
[C] im Fall, daß $R^3$ und $R^4$ zusammen einen Rest der Formel

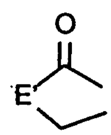

bilden,
worin
E' für ein Sauerstoff- oder Schwefelatom steht,
zunächst nach dem unter [A] und [B] aufgeführten Methoden Verbindungen der allgemeinen Formel (IX)

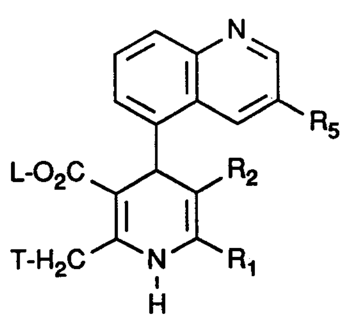

(IX)

in welcher
$R^1$, $R^2$ und $R^5$ die oben angegebene Bedeutung haben,

L für $C_1$-$C_4$-Alkyl steht

und

T für $C_1$-$C_4$-Acyloxy oder Acylthio steht,

herstellt und anschließend einen basischen oder sauren Ringschluß nach bekannten Methoden durchführt,

oder

[D] daß man im Fall, daß E für die $-CH_2$-Gruppe steht, Verbindungen der allgemeinen Formel (II) zunächst mit Acylverbindungen der allgemeinen Formel (X)

$L-CO-CH_2-R^2$ (X)

in welcher

$R^2$ die oben angegebene Bedeutung hat

und

L die oben angegebene Bedeutung von $R^1$ hat, wobei im Fall der Hydroxy-und/oder Aminofunktionen, diese gegebenenfalls in geschützter Form vorliegen,

gegebenenfalls unter Isolierung der Ylidenverbindungen der allgemeinen Formel (XI)

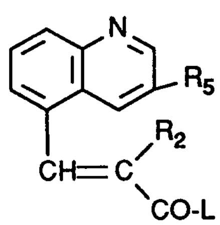

(XI)

in welcher

$R^2$, $R^5$ und L die oben angegebene Bedeutung haben

umsetzt, anschließend mit der Verbindung der Formel (XII)

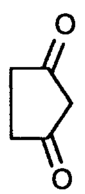

(XII)

und einer reaktiven Ammoniumverbindung, z.B. Ammoniumacetat, gegebenenfalls unter Isolierung der Zwischenprodukte der allgemeinen Formel (XIII)

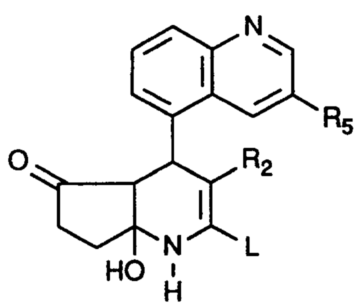

(XIII)

in welcher

$R^2$, $R^3$ und L die oben angegebene Bedeutung haben,

in inerten Lösemitteln umsetzt,
und anschließend in einem letzten Schritt, gegebenenfalls in Anwesenheit eines Hilfsmittels, Wasser abscheidet.

Die erfindungsgemäßen Verfahren können durch folgende Formelschema beispielhaft erläutert werden:

[A]

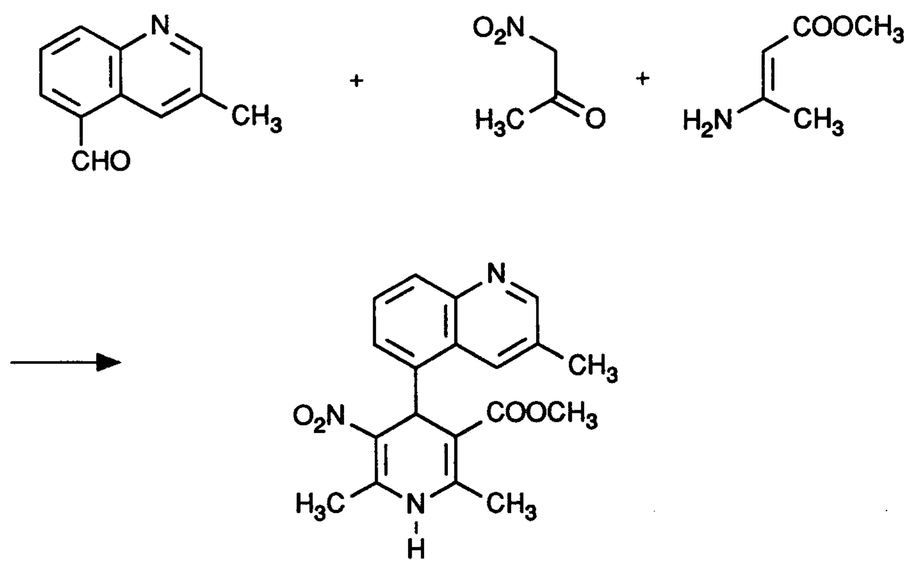

[B]

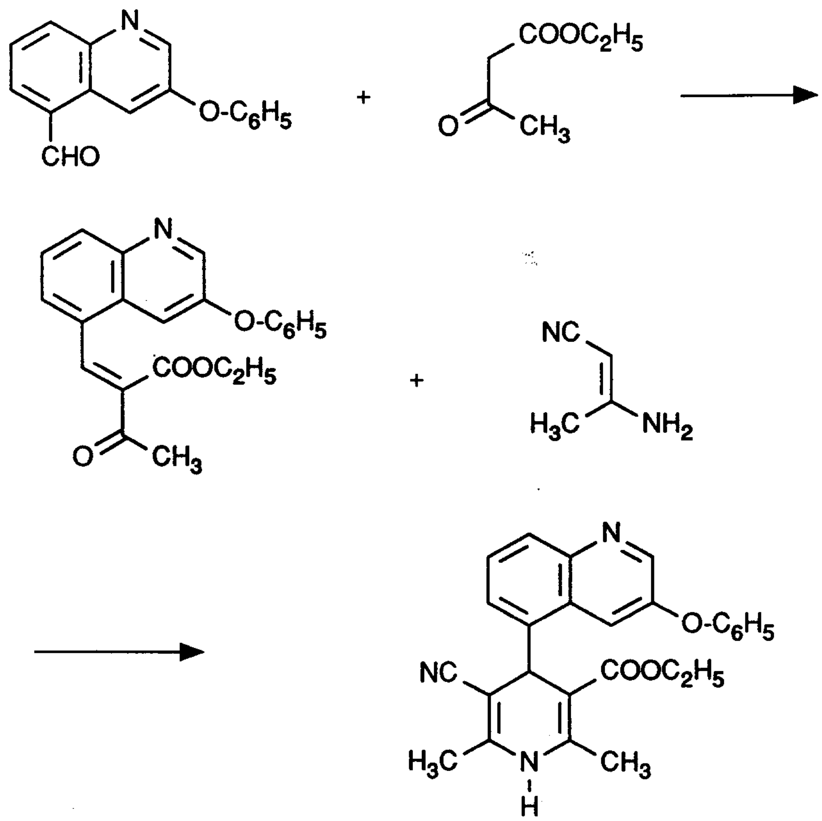

[C]

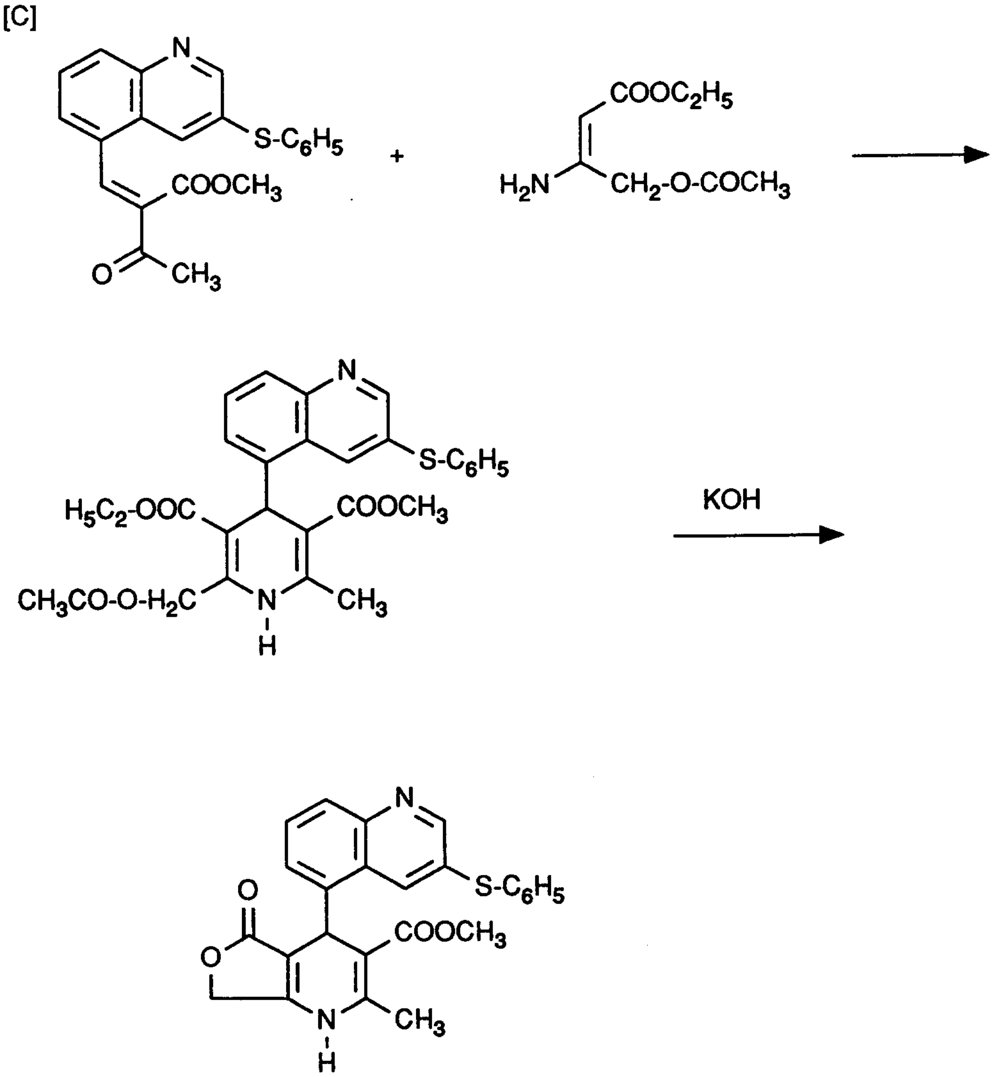

[D]

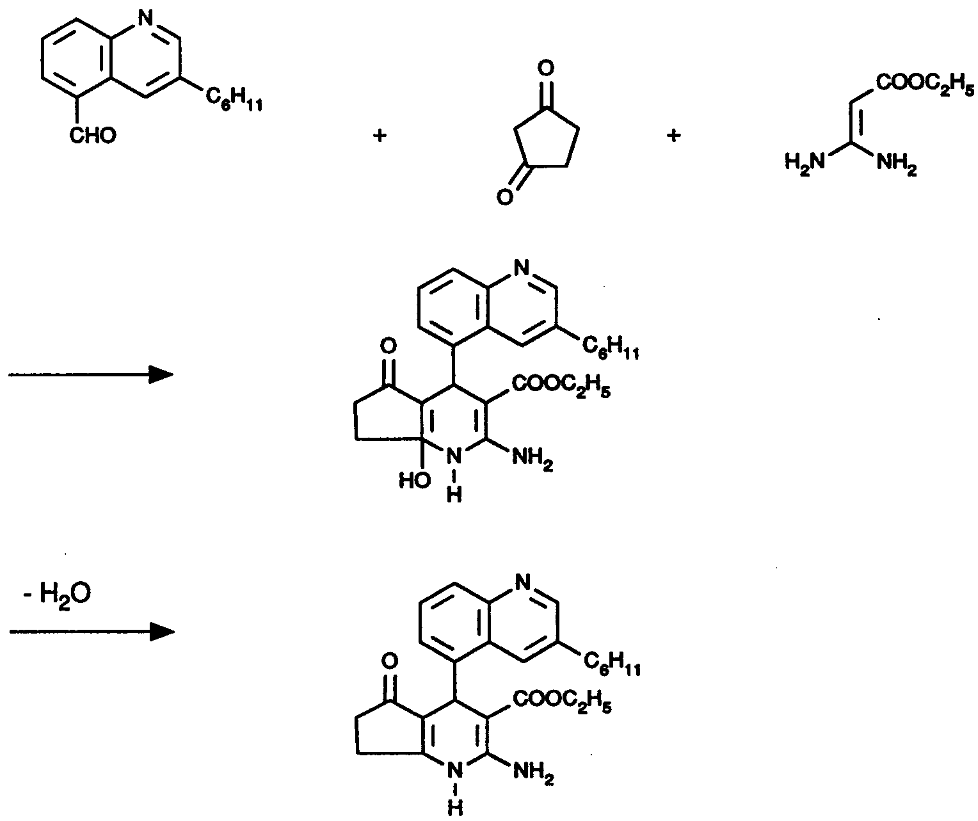


Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden.

Bevorzugt sind in Abhängigkeit von der jeweiligen Verfahrensvariante [A], [B], [C] und [D] Methanol, Isopropanol, Ethanol und n-Propanol, Acetonitril oder Tetrahydrofuran.

Die Reaktionstemperaturen können in einem größeren Bereich variiert werden. Im allgemeinen arbeitet man zwischen +10 °C und +150 °C, vorzugsweise zwischen +20 °C und +100 °C, insbesondere bei der Siedetemperatur des jeweiligen Lösemittels.

Die Umsetzung kann bei Normaldruck, aber auch bei erhöhtem oder erniedrigtem Druck (z.B. 0,5 bis 3 bar) durchgeführt werden. Im allgemeinen arbeitet man bei Normaldruck.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate, Hydroxyaminosäurederivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Verbindungen der allgemeinen Formel (II) sind neu und können in Analogie zu bekannten Verfahren bevorzugt erhalten werden, indem man 4-Amino-3-hydroxyphthalid der Formel (XIV)

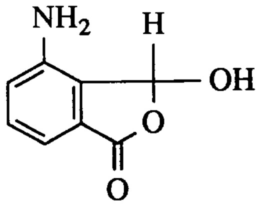

(XIV)

entweder nach Isolierung oder direkt in situ nach Hydrierung von 4-Nitro-3-hydroxyphthalid der Formel (XV)

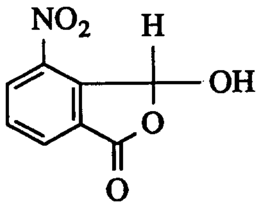

(XV)

mit Verbindungen der allgemeinen Formel (XVI)

$R^5$-$CH_2$-CHO (XVI)

zu Verbindungen der allgemeinen Formel (XVII) cyclisiert

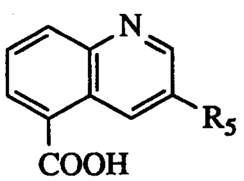

(XVII)

diese dann mit üblichen Reduktionsmitteln wie beispielsweise Lithiumaluminiumhydrid oder über ein gemischtes Anhydrid mit Natriumborhydrid in die Alkohole der allgemeinen Formel (XVIII)

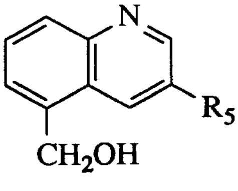

(XVIII)

überführt und diese dann entweder nach Isolierung oder direkt in situ mit Oxidationsmitteln wie beispielsweise Mangandioxid zu Verbindungen der allgemeinen Formel (II) oxidiert,
wobei
$R^5$ jeweils die oben angegebene Bedeutung hat.

Das Verfahren läßt sich durch folgendes Formelschema verdeutlichen:

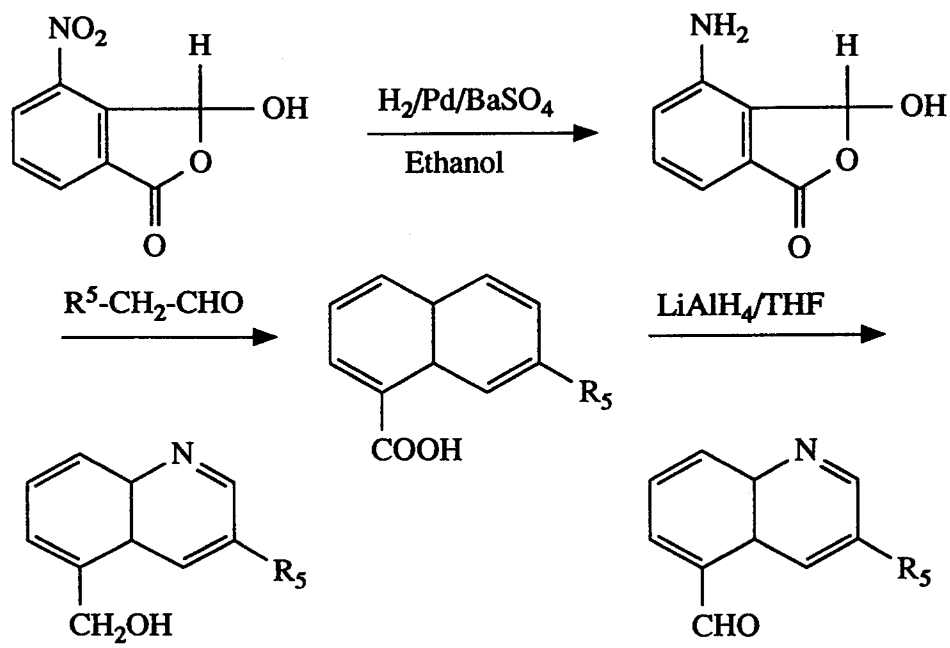


Die Acylverbindungen der allgemeinen Formel (III) und (X) sind bekannt oder können nach üblichen Methoden hergestellt werden.

Die Verbindungen der allgemeinen Formeln (V), (VI), (VIII) und (XII) sind bekannt.

Die Ylidenverbindungen (IV), (VII) und (XI) sind neu, können aber nach üblichen Methoden hergestellt werden.

Die Verbindungen der allgemeinen Formel (IX) sind neu, können aber nach bekannten Methoden hergestellt werden, indem man beispielsweise Benzylidenverbindungen der allgemeinen Formel (IV) mit Chloracetessigsäureestern und Ammoniumverbindungen umsetzt.

Die Verbindungen der allgemeinen Formel (XIII) sind neu und können wie oben beschrieben hergestellt werden.

Die vorstehenden Herstellungsverfahren sind lediglich zur Verdeutlichung angegeben. Die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur, insbesondere zeigen sie positiv inotrope Wirkungen.

Sie können deshalb in Arzneimitteln zur Beeinflussung des pathologisch veränderten Blutdrucks, als Koronartherapeutika und zur Behandlung der Herzinsuffizienz eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden. Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt, als Perfusionsmedium dient eine Krebs-Henseleit-Lösung (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l $Na_2$EDTA), deren $CaCl_2$-Gehalt 1,2 mmol/l beträgt. Als energielieferndes Substrat werden 10 mmol/l Glucose zugesetzt. vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32 °C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert. Der Perfusionsdruck wird mittels eines Druckaufnehmers, der vor dem Herzen mit dem Perfusionssystem in Verbindung steht, registiert.

Unter diesen Bedingungen zeigt eine Senkung des Perfusionsdrucks eine Koronardilatation, eine Zu- bzw. Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, wahrend in anderen Fällen die genannte obere Grenze überschritten werden muß. Im Falle der Applikation größerer Mengen kann es empfehlenswert sein, diese in mehreren Einzelgaben über den Tag zu verteilen.

<u>Ausgangsverbindungen</u>

<u>Beispiel I</u>

4-Acetoxy-2-(3-phenoxy-chinolin-5-yliden)-3-oxo-buttersäureethylester

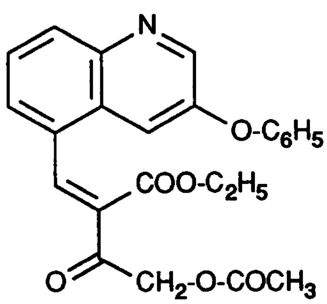

2,49 g (10 mmol) 3-Phenoxychinolin-5-aldehyd werden in 30 ml Dichlormethan mit 2,07 g (11 mmol) 4-Acetoxyacetessigsäureethylester, 0,05 ml Essigsäure und 0,1 ml Piperidin über Nacht am Wasserabscheider zum Rückflaß erhitzt. Es wird abgekühlt, zweimal mit Wasser gewaschen und eingeengt, Man erhält 4,2 g eines gelblichen Öls.

Beispiel II

Phenylthioacetaldehyd

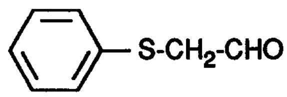

23 g (1 mol) Natrium werden in 400 ml Ethanol gelöst. Nach dem Abkühlen werden 110 g (1 mol) Thiophenol zugegeben. In diese Mischung werden 197 g (1 mol) Bromacetaldehyddiethylacetal getropft. Es wird 24 Stunden zum Rückfluß erhitzt, abgekühlt und vom ausgefallenen Salz abgesaugt. Es wird eingeengt, der Eindampfrückstand wird mit Wasser versetzt und 3 mal mit Ether extrahiert. Die vereinten Etherphasen werden mit 1 N Natronlauge und Wasser gewaschen, getrocknet und eingeengt. Der erhaltene Eindampfrückstand wird in 1,2 1 THF gelöst, mit 1 l verdünnter Salzsäure versetzt und 3 Stunden zum Rückfluß erwärmt. Es wird abgekühlt, mit Wasser verdünnt, 3 mal mit Ether extrahiert, die vereinten Etherphasen werden mit Wasser, Natriumhydrogencarbonatlösung und wieder Wasser gewaschen, getrocknet und eingeengt. Man erhält 150 g rohes Öl, welches ohne weitere Reinigung umgesetzt wird.

Beispiel III

3-Phenylthio-chinolin-5-carbonsäure

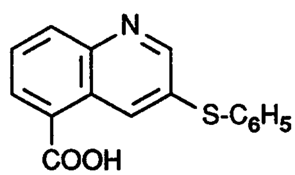

100 g (0,512 mol) 3-Hydroxy-4-nitro-phthalid werden in 500 ml Ethanol nach Zugabe von 10 g Pd/Bariumsulfat bei 3,5 bar hydriert, wobei die Temperatur auf 50 °C ansteigen darf. Es wird warm abgesaugt. In das Filtrat werden 75 g (0,5 mol) der Verbindung aus Beispiel II gegeben und über Nacht zum Rückfluß erhitzt. Das ausgefallene Produkt wird nach dem Abkühlen abgesaugt und mit Ethanol gewaschen. Man erhält 35 g einer nahezu farblosen Verbindung vom Schmelzpunkt 287-289 °C.

Beispiel IV

5-Hydroxymethyl-3-phenylthio-chinolin

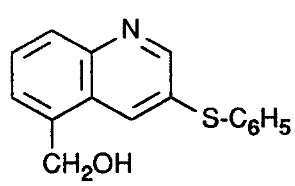

28,1 g (0,1 mol) der Verbindung aus Beispiel III werden in 800 ml trockenem THF gelöst und bei 20-30 °C tropfenweie mit 200 ml einer 1 molaren Lösung von Lithiumaluminiumhydrid in THF versetzt. Es wird 3 Stunden bei Raumtemperatur gerührt. Unter Eiskühlung werden nacheinander 8 ml Wasser und 24 ml verdünnter Kaliumhydroxid-Lösung zugetropft. Es wird 1 Stunde nachgerührt, über Celite abgesaugt und

mit THF nachgewaschen. Nach Einengen des Filtrates erhält man 27 g Rohprodukt, welches ohne Reinigung weiter umgesetzt wird.

Beispiel V

3-Phenylthio-chinolin-5-aldehyd

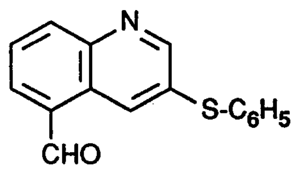

27 g der Verbindung aus Beispiel IV werden in einer Mischung von 500 ml Dimethoxyethan und 500 ml Dichlormethan gelöst und mit 165 g Mangandioxid versetzt. Es wird 2 Stunden bei Raumtemperatur gerührt, über Celite abgesaugt und mit Dichlormethan gewaschen. Nach dem Einengen wird der erhaltene Eindampfrückstand durch Flash-Chromatographie mit Toluol/Essigester-Gemischen gereinigt. Man erhält 13,7 g nahezu farbloser Kristalle vom Schmelzpunkt 46-47 °C.

Beispiel VI

3-Cyclohexyl-chinolin-5-carbonsäure

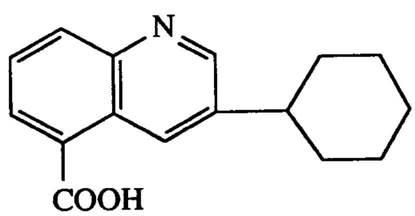

Eine Mischung von 50 g 4-Nitro-3-hydroxyphthalid in 200 ml Isopropanol wird mit 5 g Pd/Bariumsulfat bei 3,5 bar hydriert, wobei die Temperatur auf 55 °C ansteigt. Nach beendeter Hydrierung (ca. 30 Minuten) wird warm vom Katalysator abgesaugt. Das Filtrat wird mit 33 g (260 mmol) Cyclohexylacetaldehyd versetzt und nach Zugabe einer katalytischen Menge Natriummethylat 20 Stunden zum Rückfluß erhitzt. Die ausgefallenen Kristalle werden nach dem Abkühlen abgesaugt und mit Isopropanol gewaschen. Man erhält 15 g nahezu farbloses Produkt vom Schmelzpunkt über 280 °C.

Beispiel VII

3-Cyclohexyl-5-hydroxymethyl-chinolin

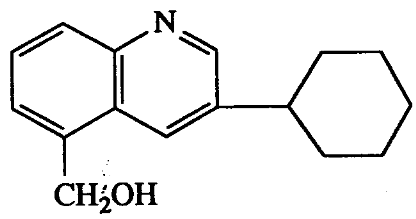

33,6 g (131,7 mmol) der Verbindung von Beispiel VI werden in 320 ml Dimethoxyethan gelöst und mit 197,6 mmol (27,6 ml) Triethylamin verrührt. Nach 30 Minuten werden 19,75 ml (197,5 mmol) Chlorkohlensäureethylester bei 20-30 °C zugetropft.

Es wird 30 Minuten nachgerührt, vom Salz abgesaugt und mit Dimethoxyethan gewaschen. Das Filtrat wird eingeengt und in einer Mischung von 320 ml Ethanol und 160 ml Wasser gelöst und bei 15-18 °C tropfenweise mit einer Lösung von 9,25 g Natriumborhydrid in 54 ml Wasser versetzt. Es wird 1 Stunde bei Raumtemperatur gerührt, vom Salz abgesaugt und mit Ethanol und Wasser gewaschen. Das Filtrat wird bis zu einem Drittel eingeengt, es wird mit Dichlormethan und Wasser versetzt und getrennt Die organische Phase wird 3 x mit Wasser gewaschen, getrocknet und eingeengt. Man erhält 26 g eines hellbraunen Öles, welches ohne weitere Reinigung eingesetzt wird.

Beispiel VIII

3-Cyclohexyl-chinolin-5-aldehyd

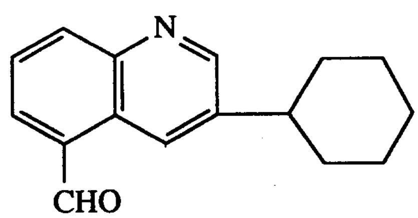

26 g der Verbindung VII werden in 260 ml Dichlormethan mit 150 g Mangandioxid versetzt. Es wird 1 Stunde gerührt, abgesaugt und mit Dichlormethan gewaschen. Das Filtrat wird eingeengt. Min erhält 20 g einer kristallinen Verbindung, die nach Umkristallisation aus Acetonitril bei 94-95 °C schmilzt.

Herstellungsbeispiele

Beispiel 1

3-Cyano-1,4-dihydro-2,6-dimethyl-4-(3-benzyl-chinolin-5-yl)-pyridin-5-carbonsäure-isopropylester

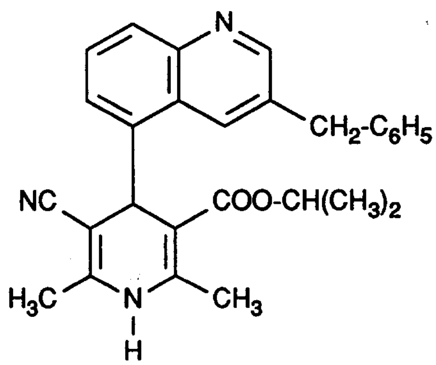

1,24 g (5 mmol) 3-Benzylchinolin-5-aldehyd werden in 20 ml Dichlormethan mit 0,72 g Acetessigsäureisopropylester, 0,025 ml Essigsäure und 0,05 ml Piperidin versetzt und über Nacht zum Sieden erhitzt. Es wird abgekühlt, mit Dichlormethan verdünnt, zweimal mit Wasser ausgeschüttelt, getrocknet und eingeengt, Das erhaltene Zwischenprodukt wird in 15 ml Isopropanol suspendiert, mit 0,41 g (5 mmol) 3-Aminocrotonsäurenitril versetzt und 20 Stunden bei 80 °C gerührt Es wird eingeengt und durch Flash-Chromatographie mit Toluol/Essigester-Gemischen gereinigt. Nach Kristallisation aus Ethanol/Ether 1:1 erhält man 1,0 g farblose Kristalle vom Schmelzpunkt 188-190 °C.

Beispiel 2

2-Methyl-4-(3-phenoxy-chinolin-5-yl)-5-oxo-1,4,5,7-tetrahydrofuro[3,4-b]pyridin-3-carbonsäureethylester

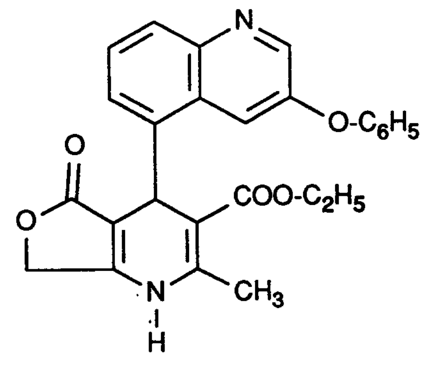

a) Herstellung der Ausgangsverbindung

2-Acetoxy-6-methyl-4-(3-phenoxy-chinolin-5-yl)-1,4-dihydropyridin-3,5-dicarbonsäure-diethylester

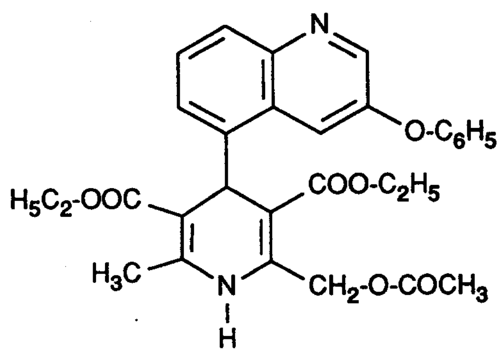

4,2 g (10 mmol) der Verbindung aus Beispiel I werden in 50 ml Isopropanol mit 1,4 g (11 mmol) 3-Aminocrotonsäureethylester 20 Stunden zum Rückfluß gekocht und eingeengt. Man erhält 5,4 g eines braunen Öles.

5,4 g der Verbindung aus Beispiel 2a werden in 60 ml Ethanol gelöst, mit 1,5 g gepulvertem Kaluimhydroxid versetzt und 1 Stunde zum Rückfluß erhitzt. Es wird abgekühlt, mit 1 N Salzsäure neutral gestellt und eingeengt. Der Eindampfrückstand wird in Dichlormethan aufgenommen, mit Wasser gewaschen, getrocknet und eingeengt. Nach Kristallisation mit Essigester wird abgesaugt und mit Essigester gewaschen. Man erhält 1,1 g einer farblosen Verbindung vom Schmelzpunkt 218-219 °C.

In Analogie zur Vorschrift des Beispiels 1 werden die in den Tabellen 1, 3, 5, 6 und 7 aufgeführten Verbindungen hergestellt:

In Analogie zur Vorschrift des Beispiels 2 werden die in den Tabellen 2 und 4 aufgeführten Verbindungen hergestellt:

Tabelle 1:

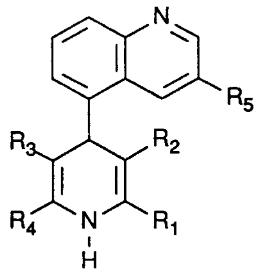

| Bsp.-Nr. | $R^1$ | $R^2$ | $R^3$ | $R^4$ | $R^5$ | F°C |
|---|---|---|---|---|---|---|
| 3 | $-CH_3$ | $-CO_2-(CH_2)_2$ — 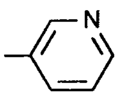 | -CN | $-CH_3$ | $-CH_2-C_6H_5$ | 205 |
| 4 | $-NH_2$ | $-CO_2CH(CH_3)_2$ | -CN | $-CH_3$ | $-CH_2-C_6H_5$ | 134-136 |
| 5 | $-CH_3$ | $-CO_2CH_3$ | -CN | $-CH_3$ | $-CH_2-C_6H_5$ | 174 |
| 6 | $-CH_3$ | $-CO_2(CH_2)_2CH_3$ | -CN | $-CH_3$ | $-CH_2-C_6H_5$ | 116 |
| 7 | $-NH_2$ | $-CO_2C_2H_5$ | -CN | $-CH_3$ | $-CH_2-C_6H_5$ | 165 |
| 8 | $-CH_3$ | $-CO_2C_2H_5$ | -CN | $-CH_3$ | $-CH_2-C_6H_5$ | 193 |
| 9 | $-CH_3$ | $-CO_2CH(CH_3)_2$ | -CN | $-CH_3$ | $-C(CH_3)_3$ | Schaum |
| 10 | $-NH_2$ | $-CO_2CH(CH_3)_2$ | -CN | $-CH_3$ | $-O-C_6H_5$ | 188-189 |
| 11 | $-NH_2$ | $-CO_2(CH_2)_2CH_3$ | -CN | $-CH_3$ | $-O-C_6H_5$ | 221 |
| 12 | $-CH_3$ | $-CO_2CH(CH_3)_2$ | -CN | $-CH_3$ | $-O-C_6H_5$ | Schaum |
| 13 | $-NH_2$ | $-CO_2CH_3$ | -CN | $-CH_3$ | $-O-C_6H_5$ | 242 |
| 14 | $-NH_2$ | $-CO_2CH(CH_3)_2$ | -CN | $-CH_3$ | $-O-C_6H_4$-p-Cl | 143-144 |
| 15 | $-CH_3$ | $-CO_2C_2H_5$ | -CN | $-CH_3$ | $-O-C_6H_4$-p-Cl | 196 |
| 16 | $-CH_3$ | $-CO_2CH(CH_3)_2$ | -CN | $-CH_3$ | $-O-C_6H_4$-p-Cl | 186 |
| 17 | $-NH_2$ | $-CO_2-(CH_2)_2-OCH_3$ | $-NO_2$ | $-CH_3$ | $-O-C_6H_5$ | 213 |

Fortsetzung Tabelle 1:

| Bsp.-Nr. | $R^1$ | $R^2$ | $R^3$ | $R^4$ | $R^5$ | F°C |
|---|---|---|---|---|---|---|
| 18 | $-NH_2$ | $-CO_2-CH(CH_3)_2$ | $-NO_2$ | $-CH_3$ | $-O-C_6H_5$ | 172 |
| 19 | $-CH_3$ | $-CO_2CH_3$ | $-NO_2$ | $-CH_3$ | $-c-C_6H_{11}$ | 270 (Zers.) |
| 20 | $-CH_3$ | $-CO_2CH_3$ | -CN | $-CH_3$ | $-c-C_6H_{11}$ | 263-265 |
| 21 | $-CH_3$ | $-CO_2CH(CH_3)_2$ | -CN | $-CH_3$ | $-c-C_6H_{11}$ | 191 |
| 22 | $-CH_3$ | $-CO_2C_2H_5$ | -CN | $-CH_3$ | $-CH(CH_3)_2$ | 120-121 |
| 23 | $-CH_3$ | $-CO_2CH_3$ | -CN | $-CH_3$ | $-CH(CH_3)_2$ | 164-166 |
| 24 | $-CH_3$ | $-CO_2C_2H_5$ | -CN | $-CH_3$ | $-c-C_6H_{11}$ | 228 |
| 25 | $-NH_2$ | $-CO_2CH(CH_3)_2$ | -CN | $-CH_3$ | $-c-C_6H_{11}$ | 212 (Zers.) |
| 26 | $-NH_2$ | $-CO_2CH_3$ | -CN | $-CH_3$ | $-CH(CH_3)_2$ | 160-161 |
| 27 | $-NH_2$ | $-CO_2C_2H_5$ | -CN | $-CH_3$ | $-c-C_6H_{11}$ | 170 |
| 28 | $-NH_2$ | $-CO_2CH_3$ | -CN | $-CH_3$ | $-c-C_6H_{11}$ | 238 |
| 29 | $-NH_2$ | $-CO_2C_2H_5$ | -CN | $-CH_3$ | $-CH(CH_3)_2$ | 141-142 |
| 30 | $-NH_2$ | $-CO_2CH(CH_3)_2$ | -CN | $-CH_3$ | $-CH(CH_3)_2$ | 223-224 |
| 31 | $-CH_3$ | $-CO_2CH(CH_3)_2$ | -CN | $-CH_3$ | $-CH(CH_3)_2$ | 172-173 |
| 32 | $-CH_3$ | $-CO_2CH_3$ | -CN | $-CH_3$ | $-S-C_6H_5$ | 207-208 |
| 33 | $-CH_3$ | $-CO_2CH(CH_3)_2$ | -CN | $-CH_3$ | $-S-C_6H_5$ | 160-162 |
| 34 | $-CH_3$ | $-CO_2CH_3$ | -CN | $-CH_3$ | $-C(CH_3)_3$ | 149-150 |
| 35 | $-NH_2$ | $-CO_2CH(CH_3)_2$ | -CN | $-CH_3$ | $-C(CH_3)_3$ | 217-218 |
| 36 | $-CH_3$ | $-CO_2C_2H_4-CN$ | -CN | $-CH_3$ | $-C(CH_3)_3$ | 184-185 |

Tabelle 2:

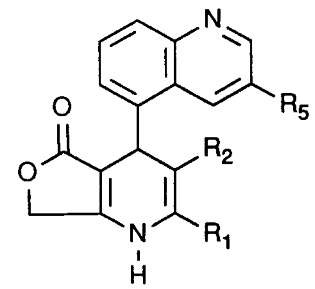

| Bsp.-Nr. | $R^1$ | $R^2$ | $R^5$ | F°C |
|---|---|---|---|---|
| 37 | $-CH_3$ | $-CO_2CH(CH_3)_2$ | $-O-C_6H_5$ | 198 |
| 38 | $-NH_2$ | $-CO_2C_2H_5$ | $-c-C_6H_{11}$ | 192 |
| 39 | $-CH_3$ | $-CO_2CH_3$ | $-c-C_6H_{11}$ | ab 262 (Zers.) |
| 40 | $-CH_3$ | $-CO_2CH(CH_3)_2$ | $-c-C_6H_{11}$ | ab 150 |
| 41 | $-NH_2$ | $-CO_2CH(CH_3)_2$ | $-c-C_6H_{11}$ | ab 192 |
| 42 | $-CH_3$ | $-CO_2CH_3$ | $-S-C_6H_5$ | 278-279 |
| 43 | $-CH_3$ | $-CO_2CH_3$ | $-CH(CH_3)_2$ | 158-160 |
| 44 | $-NH_2$ | $-CO_2CH_3$ | $-CH(CH_3)_2$ | 170-171 |
| 45 | $-CH_3$ | $-CO_2C_2H_5$ | $-CH_2-C_6H_5$ | 240 |
| 46 | $-CH_3$ | $-CO-CH_3$ | $-CH_2-C_6H_5$ | 271 |
| 47 | $-CH_3$ | $-COO(CH_2)_2-OCH_3$ | $-C(CH_3)_2$ | 195-196 |

Tabelle 3:

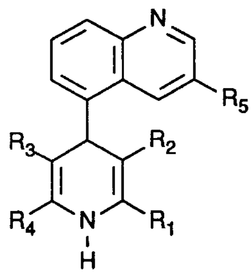

| Bsp.-Nr. | $R^1$ | $R^2$ | $R^3$ | $R^4$ | $R^5$ | F°C |
|---|---|---|---|---|---|---|
| 48 | $-NH_2$ | $-COO-CH(CH_3)_2$ | -CN | $-CH_3$ | $-CH_2$ 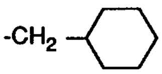 | 144 |
| 49 | $-CH_3$ | $-COOCH_3$ | -CN | $-CH_3$ | $-CH_2$ 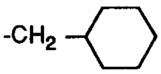 | 128-29° |
| 50 | $-CH_3$ | $-COO-(CH_2)_2$ 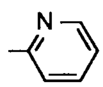 | -CN | $-CH_3$ | $-C(CH_3)_3$ | 188-89 |
| 51 | $-CH_3$ | $-COO-(CH_2)_2-N(CH_3)-CH_2$ 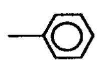 | -CN | $-CH_3$ | 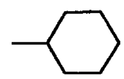 | 170-71 |
| 52 | $-C_2H_5$ | $-COO-CH(CH_3)_2$ | -CN | $-CH_3$ | 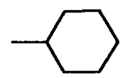 | 215-16 |
| 53 | $-NH_2$ | $-COO-CH_2$ 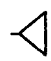 | -CN | $-CH_3$ | 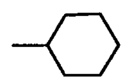 | (-)-Enant. |
| 54 | $-CF_3$ | $-COOCH(CH_3)_2$ | -CN | $-CH_3$ | 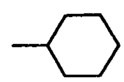 | 218 |
| 55 | $-CH_3$ | $-COO(CH_2)_2-O-CH_3$ | -CN | $-CH_3$ | 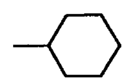 | 219 |
| 56 | $-CH_3$ | $-COOCH(CH_3)_2$ | -CN | $-CH_3$ | $-CH_2$ 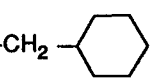 | 182 |
| 57 | $-NH_2$ | $-COO-(CH_2)_2-CH_3$ | -CN | $-CH_3$ | $-CH_2$ 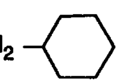 | 191 |
| 58 | $-CF_3$ | $-COOC_2H_5$ | -CN | $-CH_3$ | 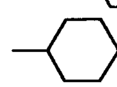 | 230 |

Fortsetzung Tabelle 3:

| Bsp.-Nr. | $R^1$ | $R^2$ | $R^3$ | $R^4$ | $R^5$ | Schmp.°C |
|---|---|---|---|---|---|---|
| 59 | $-NH_2$ | $-COOCH_3$ | -CN | $-CH_3$ | $-C(CH_3)_3$ | 174 |
| 60 | $-NH_2$ | $-COOCH_2-CH_2-OCH_3$ | -CN | $-CH_3$ | 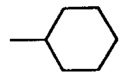 | 217 |
| 61 | $-NH_2$ | $-COO-CH_2-CH_2-CH_3$ | -CN | $-CH_3$ | 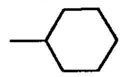 | 185 |
| 62 | $-NH_2$ | $-COO-CH(CH_3)-COOCH_3$ | -CN | $-CH_3$ | 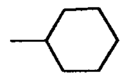 | 191 |
| 63 | $-CH_3$ | $-COO(CH_2)_2-CH_3$ | -CN | $-CH_3$ | $-CH_2$ 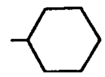 | 209 |
| 64 | $-CH_3$ | -CO-NH- 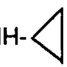 | -CN | $-CH_3$ | 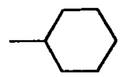 | 184 |
| 65 | $-CH_3$ | 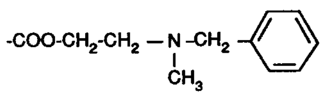 | -CN | $-CH_3$ | $-C(CH_3)_3$ | 172 |
| 66 | $-C_2H_5$ | $-COOC_2H_5$ | -CN | $-CH_3$ | 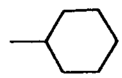 | 220 |
| 67 | $-CH_3$ | 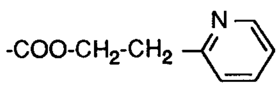 | -CN | $-CH_3$ | 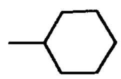 | 228 |
| 68 | $-NH_2$ | $-COO-CH_2-CH_2-$ 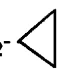 | -CN | $-CH_3$ | 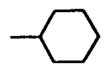 | (-)-Enant. |
| 69 | $NH_2$ | $-COO-CH_2-CH_2-OCH_3$ | CN | $-CH_3$ | $-CH_2$ 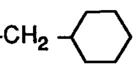 | 174 |
| 70 | $CH_3$ | $-COOC_2H_5$ | CN | $CH_3$ | $-CH_2$ 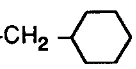 | 208 |
| 71 | $NH_2$ | $COO-CH_2-CH_2-OCH_3$ | -CN | $-CH_3$ | 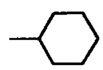 | (-)-Enant. |
| 72 | $CH_3$ | $-COO-CH_2-CH_2-CN$ | -CN | $-CH_3$ | $-CH_2$ 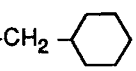 | 226 |
| 73 | $CH_3$ | $-COO-CH_2-CH_2-N-CH_2-Ph$<br>$CH_3$ | -CN | $-CH_3$ | $-CH_2$ 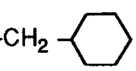 | 154 |

Fortsetzung Tabelle 3:

| Bsp.-Nr. | $R^1$ | $R^2$ | $R^3$ | $R^4$ | $R^5$ | Schmp.°C |
|---|---|---|---|---|---|---|
| 74 | $CH_3$ | $-COO-CH_2$ 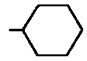 | -CN | $-CH_3$ | 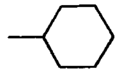 | 264-68 |
| 75 | $CH_3$ | $-COO-CH_2-CH_2-CN$ | -CN | $-CH_3$ | 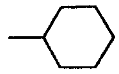 | 186 |
| 76 | $CH_3$ | $-COO-CH_2-CH_2-O-COCH_3$ | -CN | $-CH_3$ | 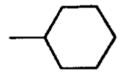 | 225 |
| 77 | $CH_3$ | -COO 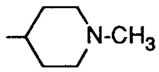 | -CN | $-CH_3$ | 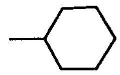 | 218 |
| 78 | $CH_3$ | 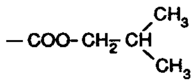 | -CN | $-CH_3$ | 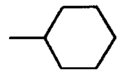 | 227-28 |
| 79 | $CH_3$ | $-COO-(CH_2)_3-CH_3$ | -CN | $-CH_3$ | 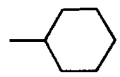 | 240-42 |
| 80 | $CH_3$ | -CO-NH 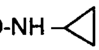 | -CN | $-CH_3$ | $-CH_2$ 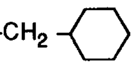 | 197-98 |
| 81 | $CH_3$ | $-CO-NH-CH_3$ | -CN | $-CH_3$ | $-CH_2$ 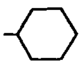 | 173-74 |
| 82 | $CH_3$ | $-CO-NH-CH_3$ | -CN | $-CH_3$ | 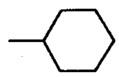 | 209 |
| 83 | $CH_3$ | $-CO-NH-(CH_2)_2-CH_3$ | -CN | $-CH_3$ | $-CH_2$ 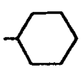 | 163 |
| 84 | $CH_3$ | $-CO-NH-CH_2-CH_3$ | -CN | $-CH_3$ | 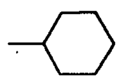 | 147 |
| 85 | $CH_3$ | $-CO-NH-(CH_2)_2-CH_3$ | -CN | $CH_3$ | 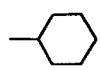 | 158-60 |
| 86 | $CH_3$ | $-COOCH_3$ | $-NO_2$ | $-CH_3$ | $-CH_2$ 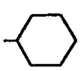 | 251-52 |
| 87 | $CH_3$ | -CO-NH 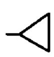 | $-NO_2$ | $-CH_3$ | $-CH_2$ 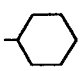 | 263-64 |
| 88 | $CH_3$ | -CO-NH 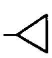 | -CN | $-CH_3$ | 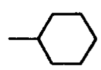 | (-)-Enant. 192-93 |

Tabelle 4:

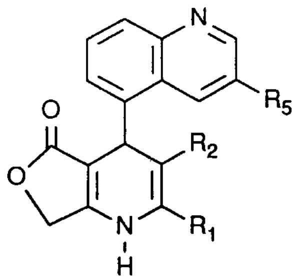

| Bsp.-Nr. | $R^1$ | $R^2$ | $R^5$ | Schmelzp. °C |
|---|---|---|---|---|
| 89 | $CH_3$ | $-COOCH_3$ | $-CH_2$ 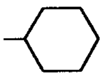 | 215-16 |
| 90 | $NH_2$ | $-COOCH-(CH_3)_2$ | -S 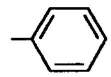 | 258 |
| 91 | $CH_3$ | $-COOCH-(CH_3)_2$ | $-CH_2$ 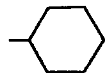 | 252 |
| 92 | $NH_2$ | $-COOCH-(CH_3)_2$ | $-CH_2$ 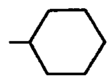 | 228 |
| 93 | $NH_2$ | $-COOC_2H_5$ | $-CH_2$ 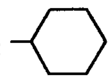 | 166 |
| 94 | $CH_3$ | -CO-NH 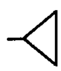 | $-CH_2$ 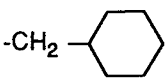 | 158-60 |
| 95 | $CH_3$ | $-CO-NH-CH_3$ | $-CH_2$ 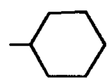 | 208-10 |
| 96 | $-CH_3$ | $-CO-NH-CH_3$ | 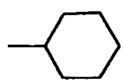 | 263 |
| 97 | $-CH_3$ | -CO-NH 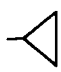 | 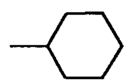 | 190 |
| 98 | $-CH_3$ | $-CO_2-(CH_2)_2$ 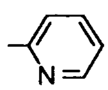 | 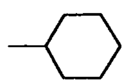 | 218 |
| 99 | $-CH_3$ | $-CO_2-CH_2$ 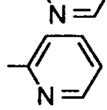 | 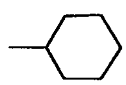 | 235 |

Tabelle 5:

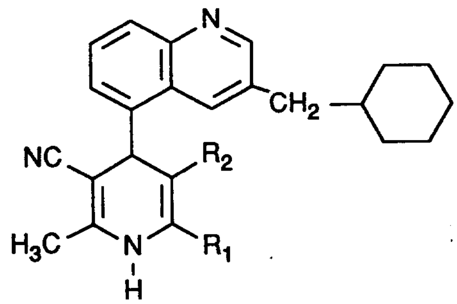

| Bsp.-Nr. | $R^1$ | $R^2$ | F°C |
|---|---|---|---|
| 100 | $-CH_3$ | $-CO_2-(CH_2)-O-CO-CH_3$ | 138-40 |
| 101 | $-CH_3$ | $-CO_2-(CH_2)_2-O-CH_3$ | 160 |
| 102 | $-CH_3$ | $CO_2-CH_2$ 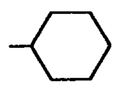 | 105 |
| 103 | $-CH_3$ | $CO_2-(CH_2)_2$ 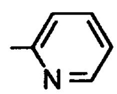 | 191 |
| 104 | $-CH_3$ | $-CO-NH-C_2H_5$ | 167-169 |
| 105 | $-NH_2$ | $-CO_2CH_3$ | 156 |

Tabelle 6:

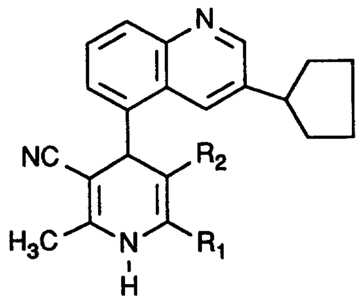

| Bsp.-Nr. | $R^1$ | $R^2$ | F°C |
|---|---|---|---|
| 106 | $-CH_3$ | -CO-NH 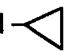 | 215-217 |
| 107 | $-CH_3$ | $-CO_2-CH(CH_3)_2$ | 203-204 |
| 108 | $-CH_3$ | $-CO_2-C_2H_5$ | 148-150 |
| 109 | $-NH_2$ | $-CO_2-CH(CH_3)_2$ | 221-223 |
| 110 | $-NH_2$ | $-CO_2-C_2H_5$ | 148-149 |

Tabelle 7:

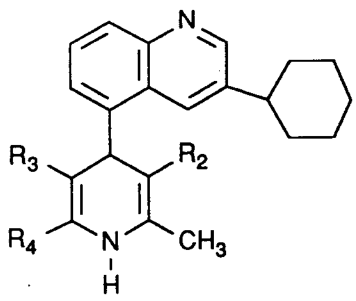

| Bsp.-Nr. | $R^2$ | $R^3$ | $R^4$ | F°C |
|---|---|---|---|---|
| 111 | -CO-NH 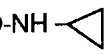 | $-NO_2$ | $-CH_3$ | 184 |
| 112 | -CO-NH 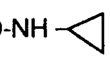 | -CN | H | 198-200 |
| 113 | -CO-NH 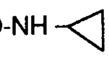 | -CN | $-CH_3$ | 185-186 |
| 114 | $-CO_2CH(CH_3)_2$ | -CN | $-CH_3$ | 148-150 |
| 115 | $-CO_2C_2H_5$ | -CN | $-CH_3$ | 160-161 |
| 116 | $-CO_2CH_3$ | -CN | $-CH_3$ | 158-160 |
| 117 | 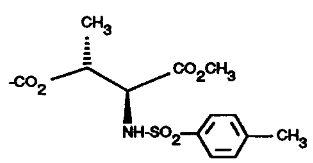 | -CN | $-CH_3$ | 250-254<br>R-Enantiomer<br>$[a]^{20}_{589}$ = 370,79<br>(C = 0,635 Dimethylformamid) |

**Patentansprüche**

**1.** 3-Chinolyl substituierte Dihydropyridine der allgemeinen Formel (I)

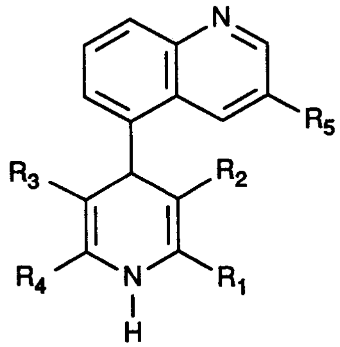

(I)

in welcher

- $R^1$ und $R^4$ — gleich oder verschieden sind und für Wasserstoff, Amino, Cyano, Formyl, Trifluormethyl oder für geradkettiges oder verzweigtes Alkyl mit bis zu 8 Kohlenstoffatomen stehen, das gegebenenfalls durch Hydroxy oder durch eine Gruppe der Formel - $NR^6R^7$, -O-CO-$R^8$, -O-$(CH_2)_a$-$OR^{8'}$ oder -O-$(CH_2)_b$-$NR^9R^{10}$ substituiert ist,
  worin
- $R^6$, $R^7$, $R^9$ und $R^{10}$ — gleich oder verschieden sind und Wasserstoff, Phenyl oder geradkettiges oder verzweigtes Alkyl mit bis zu 6 Kohlenstoffatomen bedeuten,
- $R^8$ und $R^{8'}$ — gleich oder verschieden sind und geradkettiges oder verzweigtes Alkyl mit bis zu 6 Kohlenstoffatomen bedeuten,
  und
- a und b — gleich oder verschieden sind und eine Zahl 2, 3, 4 oder 5 bedeuten,
- $R^2$ — für eine Gruppe der Formel - CO-$NR^{11}R^{12}$ oder -CO-A-$R^{13}$ steht,
  worin
- $R^{11}$ und $R^{12}$ — gleich oder verschieden sind und Wasserstoff, einen geradkettigen, verzweigten oder cyclischen, gesättigten oder ungesättigten Kohlenwasserstoffrest mit bis zu 8 Kohlenstoffatomen bedeuten, der gegebenenfalls durch Halogen, Hydroxy, Cyano oder durch Aryl, Aryloxy, Arylthio mit jeweils 6 bis 10 Kohlenstoffatomen oder durch einen 5-bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder
  Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind,
  oder
- $R^{11}$ und $R^{12}$ — gemeinsam unter Einbezug des Stickstoffatoms einen 3-bis 8-gliedrigen, gesättigen oder ungesättigten Heterocyclus bilden, der gegebenenfalls durch ein Sauerstoffatom oder durch einen Rest der Formel $S(O)_d$, - CO- oder -$NR^{15}$ unterbrochen sein kann,
  worin
- d — eine Zahl 0, 1 oder 2 bedeutet,
- $R^{15}$ — Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl mit jeweils bis zu 8 Kohlenstoffatomen, Halogenalkyl, Halogenalkoxy oder Halogenalkylthio mit jeweils bis zu 4 Kohlenstoffatomen substituiert ist, oder

einen cyclischen, gesättigten oder ungesättigten, geradkettigen oder verzweigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkoxy oder Alkylthio mit jeweils bis zu 4 Kohlenstoffatomen, Halogen, Aryl mit 6 bis 10 Kohlenstoffatomen, einen 5-bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das seinerseits durch Aryl mit 6 bis 10 Kohlenstoffatomen substituiert sein kann,

A eine direkte Bindung oder ein Sauerstoffatom bedeutet,

$R^{13}$ Wasserstoff oder Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeutet, wobei die Cyclen gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder

einen cyclischen, geradkettigen oder verzweigten, gesättigten oder ungesättigten Kohlenwasserstoffrest mit bis zu 10 Kohlenstoffatomen bedeutet, der gegebenenfalls bis zu 3-fach gleich oder verschieden durch Sauerstoff oder durch - CO-, -CO-NH-, -O-CO-, -CO-O-, -NH-CO-, $-SO_2-NH-$, $-NH-SO_2-$, $-S(O)_e-$oder$-NR^{16}-$unterbrochen ist,

worin

e die oben angegebene Bedeutung von d hat und mit dieser gleich oder verschieden ist,

$R^{16}$ die oben angegebene Bedeutung von $R^{15}$ hat und mit dieser gleich oder verschieden ist,

oder der Kohlenwasserstoffrest gegebenenfalls bis zu 3-fach gleich oder verschieden durch Aryliden mit 6 bis 10 Kohlenstoffatomen oder heterocyclische Reste der Formeln

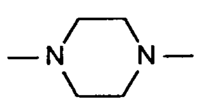 , 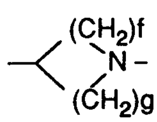 , 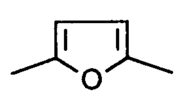 oder 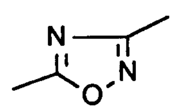

unterbrochen ist,

worin

f und g gleich oder verschieden sind und eine Zahl 1 oder 2 bedeuten,

und wobei Aryliden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein kann,

und der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, $-O-NO_2$, geradkettiges oder verzweigtes Alkylthio, Alkoxy oder Acyloxy mit jeweils bis zu 8 Kohlenstoffatomen oder durch Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, wobei

die Cyclen ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder

der Kohlenwasserstoffrest gegebenenfalls durch eine Gruppe der Formel $-CO_2-R^{17}$, $-CONR^{18}R^{19}$, $-NR^{20}R^{21}$, $-NH-SO_2-X$ und/oder $-NR^{22}-CO_2R^{23}$ substituiert ist,

worin

$R^{17}$ die oben angegebene Bedeutung von $R^{15}$ hat und mit dieser gleich oder verschieden ist

und

$R^{18}$, $R^{19}$, $R^{20}$, $R^{21}$, $R^{22}$ und $R^{23}$ die oben angegebene Bedeutung von $R^{11}$ und $R^{12}$ haben und mit diesen gleich oder verschieden sind,

und

X Phenyl bedeutet, das gegebenenfalls durch Methyl substituiert ist,

$R^3$ für Cyano, Nitro oder Formyl steht,

oder

$R^3$ und $R^4$ gemeinsam einen Rest der Formel

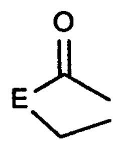

bilden,

worin

E ein Sauerstoff-oder Schwefelatom oder die $-CH_2$-Gruppe bedeutet,

$R^5$ für einen cyclischen, geradkettigen oder verzweigten, gesättigten oder ungesättigten Kohlenwasserstoffrest mit bis zu 12 Kohlenstoffatomen steht, der gegebenenfalls bis zu 2-fach gleich oder verschieden durch Sauerstoff oder Schwefel unterbrochen ist,

und der gegebenenfalls bis zu 3-fach gleich oder verschieden durch Cycloalkyl mit 3 bis 8 Kohlenstoffatomen, geradkettiges oder verzweigtes Acyloxy mit bis zu 4 Kohlenstoffatomen, Halogen, Nitro, Cyano, Hydroxy oder durch Aryl, Aryloxy oder Arylthio mit jeweils 6 bis 10 Kohlenstoffatomen oder durch einen 5-bis 7-gliedrigen, gesättigten oder ungesättigen gegebenenfalls kondensierten Heterocyclus mit bis zu 5 Heteroatomen aus der Reihe S, N oder O substituiert ist, wobei die Cyclen ihrerseits bis zu 3-fach gleich oder verschieden durch Halogen, Cyano, Nitro, Hydroxy oder durch geradkettiges oder verzweigtes Alkyl oder Alkoxy mit jeweils bis zu 4 Kohlenstoffatomen, Trifluormethyl, Trifluormethoxy, Trifluormethylthio oder durch eine Gruppe der Formel $-NR^{24}R^{25}$ substituiert sein können,

worin

$R^{24}$ und $R^{25}$ die oben angegebene Bedeutung von $R^{11}$ und $R^{12}$ haben und mit diesen gleich oder verschieden sind,

oder der Kohlenwasserstoffrest gegebenenfalls durch eine Gruppe der Formel $-CO_2-R^{26}$, $-CONR^{27}R^{28}$, $-NR^{29}R^{30}$, $-NR^{31}-CO_2R^{32}$ oder $-NR^{33}-SO_2R^{34}$ substituiert ist,

worin

$R^{26}$ die oben angegebene Bedeutung von $R^{15}$ hat und mit dieser gleich oder verschieden ist

und

$R^{27}$, $R^{28}$, $R^{29}$, $R^{30}$, $R^{31}$, $R^{32}$, $R^{33}$ und $R^{34}$ die oben angegebene Bedeutung von $R^{11}$ und $R^{12}$ haben und mit diesen gleich oder verschieden sind,

oder

$R^5$ für einen 5-bis 7-gliedrigen, gesättigten oder ungesättigten Heterocyclus mit bis zu 4 Heteroatomen aus der Reihe S, N oder O steht, der gegebenenfalls bis zu 3-fach gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono-oder - Dialkylamino substituiert ist,

oder

$R^5$ für eine Gruppe der Formel D-$R^{35}$ steht,

worin

D die CO-oder - $S(O)_h$-Gruppe oder ein Sauerstoffatom bedeutet,

worin

h eine Zahl 0, 1 oder 2 bedeutet,

und

$R^{35}$ Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeutet, die gegebenenfalls bis zu 3-fach gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono-oder -Dialkylamino substituiert sind,

oder

$R^{35}$ Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel unterbrochen ist,

und der gegebenenfalls durch Halogen, Aryl, Aryloxy oder Arylthio mit jeweils 6 bis 10 Kohlenstoffatomen oder durch einen 5-bis 7-gliedrigen, gesättigten oder ungesättigen Heterocyclus mit bis zu 3 Heteroatomen aus der Reihe S, N oder O substituiert ist, wobei die Cyclen ihrerseits durch Halogen, Trifluormethyl, Methyl, Methoxy, Nitro oder Methylthio substituiert sein können,

oder

durch eine Gruppe der Formel - $NR^{36}R^{37}$ substituiert ist,

worin

$R^{36}$ und $R^{37}$ die oben angegebene Bedeutung von $R^{11}$ und $R^{12}$ haben und mit diesen gleich oder verschieden sind,

und deren Salze,

mit der Maßgabe, daß $R^5$ nicht für gegebenenfalls halogensubstituiertes Pyridyl oder Thienyl stehen darf.

**2.** Verbindungen der allgemeinen Formel (I) gemaß Anspruch 1, in welcher

$R^1$ und $R^4$ gleich oder verschieden sind und für Wasserstoff, Amino, Cyano,

Formyl, Trifluormethyl oder für geradkettiges oder verzweigtes Alkyl mit bis zu 4 Kohlenstoffatomen stehen, das gegebenenfalls durch Hydroxy, durch eine Gruppe der Formel - $NR^6R^7$, -O-CO-$R^8$, -O-$(CH_2)_a$-$OR^{8'}$ oder -O-$(CH_2)_{b'}$-$NR^9R^{10}$ substituiert ist,

worin

$R^6$, $R^7$, $R^9$ und $R^{10}$ gleich oder verschieden sind und Wasserstoff oder geradkettiges oder verzweigtes Alkyl mit bis zu 4 Kohlenstoffatomen bedeuten,

$R^8$ und $R^{8'}$ gleich oder verschieden sind und geradkettiges oder verzweigtes Alkyl mit bis zu 4 Kohlenstoffatomen bedeuten,

und

a und b gleich oder verschieden sind und eine Zahl 2, 3, 4 oder 5 bedeuten,

$R^2$ für eine Gruppe der Formel - CO-$NR^{11}R^{12}$ oder -CO-A-$R^{13}$ steht,

worin

$R^{11}$ und $R^{12}$ gleich oder verschieden sind und Wasserstoff, einen geradkettigen, verzweigten oder cyclischen, gesättigten oder ungesättigten Kohlenwasserstoffrest mit bis zu 6 Kohlenstoffatomen bedeuten, der gegebenenfalls durch Fluor, Chlor, Hydroxy, Phenyl oder Pyridyl substituiert ist, wobei die Cyclen ihrerseits durch Fluor, Chlor oder durch Alkyl, Alkoxy, Alkylthio oder Alkoxycarbonyl mit jeweils bis zu 2 Kohlenstoffatomen, Trifluormethyl oder Trifluormethoxy substituiert sein können, oder

Phenyl oder Pyridyl bedeuten, die gegebenenfalls durch Fluor, Chlor oder durch Alkyl, Alkoxy, Alkylthio oder Alkoxycarbonyl mit jeweils bis zu 2 Kohlenstoffatomen, Trifluormethyl oder Trifluormethoxy substituiert sind,

oder

$R^{11}$ und $R^{12}$ gemeinsam und Einbezug des Stickstoffatoms einen 3-bis 8-gliedrigen, gesättigen oder ungesättigten Heterocyclus bilden, der gegebenenfalls durch ein Sauerstoffatom oder durch einen Rest der Formel $S(O)_d$, - CO- oder -$NR^{15}$ unterbrochen sein kann,

worin

d eine Zahl 0, 1 oder 2 bedeutet,

$R^{15}$ Wasserstoff oder Phenyl bedeutet, das gegebenenfalls durch Fluor, Chlor, Methyl, Ethyl, Methoxy, Ethoxy, Trifluormethyl oder Trifluormethoxy substituiert ist, oder

einen cyclischen, gesättigten oder ungesättigten, geradkettigen oder verzweigten Kohlenwasserstoffrest mit bis zu 4 Kohlenstoffatomen bedeutet, der gegebenenfalls durch Fluor, Chlor, Phenyl oder durch einen 5- bis 7-gliedrigen, gesättigten oder ungesättigten Heterocyclus mit bis zu 3 Heteroatomen aus der Reihe S, N oder O substituiert ist, wobei die Cyclen ihrerseits durch Fluor, Chlor, Methyl, Methoxy, Methylthio, Trifluormethyl oder Trifluormethoxy substituiert sein können,

A eine direkte Bindung oder ein Sauerstoffatom bedeutet,

$R^{13}$ Wasserstoff, Phenyl oder Pyridyl bedeutet, die gegebenenfalls durch Fluor, Chlor, Methyl, Methoxy, Methylthio, Trifluormethyl oder Trifluormethoxy substituiert sind, oder

einen cyclischen, geradkettigen oder verzweigten, gesättigten oder ungesättigten Kohlenwasserstoffrest mit bis zu 8 Kohlenstoffatomen bedeutet, der gegebenenfalls bis zu 2-fach gleich oder verschieden durch Sauerstoff oder durch - CO-, -CO-NH-, -O-CO-, -CO-O-, -NH-CO-, -$SO_2$-NH-, -NH-$SO_2$-, -$S(O)_e$-oder-$NR^{16}$-unterbrochen ist,

worin

e die oben angegebene Bedeutung von d hat und mit dieser gleich oder verschieden ist,

$R^{16}$ die oben angegebene Bedeutung von $R^{15}$ hat und mit dieser

gleich oder verschieden ist,

oder der Kohlenwasserstoffrest gegebenenfalls bis zu 2-fach gleich oder verschieden durch Aryliden mit 6 bis 10 Kohlenstoffatomen oder heterocyclische Reste der Formeln

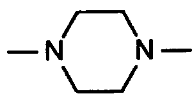 , 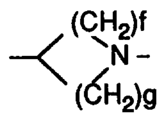 , 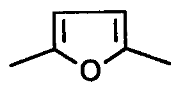 oder 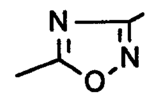

unterbrochen ist,

worin

f und g — gleich oder verschieden sind und eine Zahl 1 oder 2 bedeuten,

und der Kohlenwasserstoffrest gegebenenfalls bis zu 2-fach gleich oder verschieden durch Cyclopropyl, Cyclobutyl, Cyclopentyl, Cyclohexyl, Fluor, Chlor, Nitro, Cyano, Hydroxy, $-O-NO_2$, geradkettiges oder verzweigtes Alkylthio, Alkoxy oder Acyloxy mit jeweils bis zu 4 Kohlenstoffatomen oder durch Phenyl, Phenoxy, Phenylthio oder Pyridyl substituiert ist, wobei die Cyclen ihrerseits durch Fluor, Chlor, Cyano, Methyl, Methoxy, Methylthio, Trifluormethyl oder Trifluormethoxy substituiert sein können, oder der Kohlenwasserstoffrest gegebenenfalls durch eine Gruppe der Formel $-CO_2-R^{17}$, $-CONR^{18}R^{19}$, $-NR^{20}R^{21}$, $-NH-SO_2-X$ und/oder $-NR^{22}-CO_2R^{23}$ substituiert ist,

worin

$R^{17}$ — die oben angegebene Bedeutung von $R^{15}$ hat und mit dieser gleich oder verschieden ist

und

$R^{18}$, $R^{19}$, $R^{20}$, $R^{21}$, $R^{22}$ und $R^{23}$ — die oben angegebene Bedeutung von $R^{11}$ und $R^{12}$ haben und mit diesen gleich oder verschieden sind,

und

X — Phenyl bedeutet, das gegebenenfalls durch Methyl substituiert ist,

$R^3$ — für Cyano, Nitro oder Formyl steht,

oder

$R^3$ und $R^4$ gemeinsam einen Rest der Formel

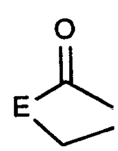

bilden,

worin

E — ein Sauerstoff-oder Schwefelatom oder die $-CH_2$-Gruppe bedeutet,

$R^5$ — für einen cyclischen, geradkettigen oder verzweigten, gesättigten oder ungesättigten Kohlenwasserstoffrest mit bis zu 10 Kohlenstoffatomen steht, der gegebenenfalls durch Sauerstoff oder Schwefel unterbrochen ist,

und der gegebenenfalls bis zu 2-fach gleich oder verschieden durch Cyclopropyl, Cyclobutyl, Cyclopentyl, Cyclohexyl, Fluor, Chlor, Brom, Acyloxy mit bis zu 2 Kohlenstoffatomen, Cyano, Hydroxy oder durch Phenyl, Phenyloxy, Phenylthio oder durch einen 5-bis 7-gliedrigen, gesättigten oder ungesättigen Heterocyclus mit bis

zu 3 Heteroatomen aus der Reihe S, N oder O substituiert ist, wobei die Phenyl-und die Hetero-Cyclen ihrerseits bis zu 2-fach gleich oder verschieden durch Fluor, Chlor, Brom, Hydroxy, Methyl, Ethyl, Methoxy, Ethoxy, Trifluormethyl, Trifluormethoxy oder Trifluormethylthio oder durch eine Gruppe der Formel - $NR^{24}R^{25}$ substituiert sein können,

worin

$R^{24}$ und $R^{25}$ die oben angegebene Bedeutung von $R^{11}$ und $R^{12}$ haben und mit diesen gleich oder verschieden sind,

oder der Kohlenwasserstoffrest gegebenenfalls durch eine Gruppe der Formel - $CO_2$-$R^{26}$, - $CONR^{27}R^{28}$, - $NR^{29}R^{30}$, - $NR^{31}$-$CO_2R^{32}$ oder -$NR^{33}$-$SO_2R^{34}$ substituiert ist,

worin

$R^{26}$ die oben angegebene Bedeutung von $R^{15}$ hat und mit dieser gleich oder verschieden ist

und

$R^{27}$, $R^{28}$, $R^{29}$, $R^{30}$, $R^{31}$, $R^{32}$, $R^{33}$ und $R^{34}$ die oben angegebene Bedeutung von $R^{11}$ und $R^{12}$ haben und mit diesen gleich oder verschieden sind,

oder

$R^5$ für einen 5-bis 7-gliedrigen, gesättigten oder ungesättigten Heterocyclus mit bis zu 4 Heteroatomen aus der Reihe S, N oder O steht, der gegebenenfalls bis zu 2-fach gleich oder verschieden durch Fluor, Chlor, Brom, Cyano, Nitro oder durch Alkyl, Alkoxy, Alkylthio, Alkoxycarbonyl, Halogenalkyl, Halogenalkoxy oder Halogenalkylthio mit jeweils bis zu 2 Kohlenstoffatomen, Amino oder durch $C_1$-$C_4$-Mono-oder - Dialkylamino substituiert ist,

oder

$R^5$ für eine Gruppe der Formel D-$R^{35}$ steht,

worin

D die CO-oder - $S(O)_h$-Gruppe oder ein Sauerstoffatom bedeutet,

worin

h eine Zahl 0, 1 oder 2 bedeutet,

und

$R^{35}$ Phenyl oder einen 5-bis 7-gliedrigen, gesättigten oder ungesättigten Heterocyclus mit bis zu 3 Heteroatomen aus der Reihe S, N oder O bedeutet, die gegebenenfalls bis zu 2-fach gleich oder verschieden durch Fluor, Chlor, Brom, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen, Amino oder durch $C_1$-$C_4$-Mono-oder - Dialkylamino substituiert sind,

oder

$R^{35}$ Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel unterbrochen ist, und der gegebenenfalls durch Fluor, Chlor, Phenyl, Phenoxy oder Phenylthio substituiert sein kann,

oder

durch eine Gruppe der Formel - $NR^{36}R^{37}$ substituiert ist,

worin

$R^{36}$ und $R^{37}$ die oben angegebene Bedeutung von $R^{11}$ und $R^{12}$ haben

und mit dieser gleich oder verschieden sind,

und deren Salze,

mit der Maßgabe, daß $R^5$ nicht für gegebenenfalls halogensubstituiertes Pyridyl oder Thienyl stehen darf.

**3.** Verbindungen der allgemeinen Formel (I) gemaß Anspruch 1, in welcher

$R^1$ und $R^4$ gleich oder verschieden sind und für Wasserstoff, Amino, Trifluormethyl, Methyl oder Ethyl stehen,

$R^2$ für eine Gruppe der Formel - $CO\text{-}NR^{11}R^{12}$ oder $\text{-}CO\text{-}A\text{-}R^{13}$ steht,

worin

$R^{11}$ und $R^{12}$ gleich oder verschieden sind und Wasserstoff, einen geradkettigen, verzweigten oder cyclischen, gesättigten oder ungesättigten Kohlenwasserstoffrest mit bis zu 6 Kohlenstoffatomen bedeuten, oder

Phenyl bedeuten, das gegebenenfalls durch Fluor, Chlor, Methyl oder Methoxy substituiert ist,

A eine direkte Bindung oder ein Sauerstoffatom bedeutet,

$R^{13}$ Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel oder durch - CO-NH-, -O-CO-, -CO-O-, -NH-CO-, $\text{-}SO_2\text{-}NH\text{-}$, $\text{-}NH\text{-}SO_2\text{-}$ oder $\text{-}NR^{16}$-unterbrochen ist, worin

$R^{16}$ Wasserstoff, geradkettiges oder verzweigtes Alkyl mit bis zu 4 Kohlenstoffatomen bedeutet,

oder der Kohlenwasserstoffrest durch heterocyclische Reste der Formeln

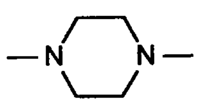

oder

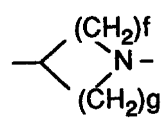

unterbrochen ist,

worin

f und g gleich oder verschieden sind und eine Zahl 1 oder 2 bedeuten,

und der Kohlenwasserstoffrest gegebenenfalls durch Cyclopropyl, Cyclobutyl, Cyclopentyl, Cyclohexyl, Fluor, Chlor, Nitro, Cyano, Hydroxy oder durch Phenyl, Phenoxy, Phenylthio oder Pyridyl substituiert ist, die ihrerseits durch Fluor, Chlor, Methyl, Methoxy, Methylthio, Trifluormethyl oder Trifluormethoxy substituiert sein können, oder der Kohlenwasserstoffrest gegebenenfalls durch eine Gruppe der Formel $\text{-}CO_2R^{17}$, $\text{-}CONR^{18}R^{19}$, - $NR^{20}R^{21}$, $\text{-}NH\text{-}SO_2\text{-}X$ und/oder $\text{-}NR^{22}\text{-}CO_2R^{23}$ substituiert ist,

worin

$R^{20}$ und $R^{21}$ gleich oder verschieden sind und Wasserstoff, einen geradkettigen, verzweigten oder cyclischen, gesättigten oder ungesättigten Kohlenwasserstoffrest mit bis zu 6 Kohlenstoffatomen bedeuten, das gegebenenfalls durch Fluor, Chlor, Pyridyl oder Phenyl substituiert ist, das seinerseits durch Fluor, Chlor, Methyl oder Methoxy substituiert sein kann, oder

Phenyl bedeuten, das gegebenenfalls durch Fluor, Chlor, Methyl oder Methoxy substituiert ist,

oder

$R^{20}$ und $R^{21}$ gemeinsam unter Einbezug des Stickstoffatoms einen 5-bis 6-gliedrigen, gesättigen oder ungesättigten Heterocyclus bilden, der gegebenenfalls bis zu 2 weitere Heteroatome aus der Reihe S, N oder O enthalten kann und der gegebenenfalls auch geradkettiges oder verzweigtes Alkyl mit bis zu 4 Kohlenstoffatomen, Benzyl oder Phenyl substituiert ist,

X Phenyl bedeutet, das gegebenenfalls durch Methyl substituiert ist.

$R^{17}$, $R^{18}$, $R^{19}$, $R^{22}$ und $R^{23}$ gleich oder verschieden sind und Wasserstoff, geradkettiges oder verzweigtes Alkyl mit bis zu 6 Kohlenstoffatomen bedeuten, das gegebenenfalls durch Phenyl substituiert ist, oder Phenyl bedeuten, das gegebenenfalls durch Fluor, Chlor oder Brom substituiert ist,

$R^3$ für Cyano, Nitro oder Formyl steht,

oder

$R^3$ und $R^4$ gemeinsam einen Rest der Formel

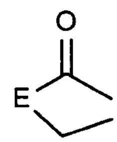

bilden,

worin

E ein Sauerstoff-oder Schwefelatom oder die - $CH_2$-Gruppe bedeutet,

$R^5$ für einen cyclischen, geradkettigen oder verzweigten, gesättigten oder ungesättigten Kohlenwasserstoffrest mit bis zu 8 Kohlenstoffatomen steht, der gegebenenfalls durch Sauerstoff oder Schwefel unterbrochen ist, und der gegebenenfalls durch Cyclopropyl, Cyclobutyl, Cyclopentyl, Cyclohexyl, Fluor, Chlor, Nitro, Cyano, Hydroxy oder durch Phenyl, Phenoxy, Phenylthio oder durch einen 5-bis 7-gliedrigen, gesättigten oder ungesättigen Heterocyclus mit bis zu 2 Heteroatomen aus der Reihe S, N oder O substituiert ist, wobei die Phenyl- und die Hetero-Cyclen ihrerseits bis zu 2-fach gleich oder verschieden durch Fluor, Chlor, Methyl, Methoxy, Trifluormethyl oder Trifluormethoxy oder durch eine Gruppe der Formel - $NR^{24}R^{25}$ substituiert sein können,

worin

$R^{24}$ und $R^{25}$ die oben angegebene Bedeutung von $R^{11}$ und $R^{12}$ haben und mit dieser gleich oder verschieden sind,

oder der Kohlenwasserstoffrest gegebenenfalls durch eine Gruppe der Formel - $CO_2$-$R^{26}$, - $CONR^{27}R^{28}$, - $NR^{29}R^{30}$, - $NR^{31}$-$CO_2R^{32}$ oder -$NR^{33}$-$SO_2R^{34}$ substituiert ist,

worin

$R^{26}$ Alkyl mit 1-4 C-Atomen oder Phenyl bedeutet,

und

$R^{27}$, $R^{28}$, $R^{29}$, $R^{30}$, $R^{31}$, $R^{32}$, $R^{33}$ und $R^{34}$ die oben angegebene Bedeutung von $R^{11}$ und $R^{12}$ haben und mit diesen gleich oder verschieden sind,

oder

$R^5$ für einen 5-bis 6-gliedrigen, gesättigten oder ungesättigten Heterocyclus mit bis zu 2 Heteroatomen aus der Reihe S, N oder O steht, der gegebenenfalls durch Fluor, Chlor, Methyl, Methoxy, Methylthio oder Trifluormethyl substituiert ist,

oder

$R^5$ für eine Gruppe der Formel D-$R^{35}$ steht,

worin

D die - $S(O)_h$-Gruppe oder ein Sauerstoffatom bedeutet, worin

h eine Zahl 0, 1 oder 2 bedeutet, und

$R^{35}$ Phenyl bedeutet, das gegebenenfalls durch Fluor, Chlor, Brom, Methyl, Methoxy, Methylthio,Trifluormethyl, Amino oder durch $C_1$-$C_2$-Mono-oder - Dialkylamino substituiert ist, oder

$R^{35}$ Wasserstoff, einen cyclischen, geradkettigen oder verzweigten, gesättigten oder ungesättigten Kohlenwasserstoffrest mit bis zu 6 Kohlenstoffatomen bedeutet, der gegebenenfalls durch Sauerstoff oder Schwefel unterbrochen ist, und der gegebenenfalls durch Fluor, Chlor oder Phenyl substituiert ist, oder durch eine Gruppe der Formel - $NR^{36}R^{37}$ substituiert ist worin

$R^{36}$ und $R^{37}$ die oben angegebene Bedeutung von $R^{11}$ und $R^{12}$ haben und mit dieser gleich oder verschieden sind,

und deren Salze,
mit der Maßgabe, daß $R^5$ nicht für gegebenenfalls halogensubstituiertes Pyridyl oder Thienyl stehen darf.

**4.** Verbindungen der allgemeinen Formel (I) gemaß Anspruch 1, in welcher

$R^1$ für Wasserstoff, Methyl, Ethyl, Trifluormethyl oder Amino steht,

$R^4$ für Wasserstoff, Methyl oder Amino steht,

$R^2$ für eine Gruppe der Formel -CO-$NR^{11}R^{12}$ oder -CO-A-$R^{13}$ steht, worin

$R^{11}$ und $R^{12}$ gleich oder verschieden sind und Wasserstoff oder einen gesättigten oder ungesättigten geradkettigen, verzweigten oder cyclischen Kohlenwasserstoffrest mit bis zu 6 Kohlenstoffatomen bedeuten,

A eine direkte Bindung oder ein Sauerstoffatom bedeutet,

$R^{13}$ Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel oder durch -O-CO-, -CO-O- oder -$NR^{16}$ unterbrochen ist, worin

$R^{16}$ Wasserstoff oder Methyl bedeutet,

und der Kohlenwasserstoffrest gegebenenfalls durch Cyclopropyl, Cyclobutyl, Cyclopentyl, Cyclohexyl, Fluor, Chlor, Nitro, Cyano, Hydroxy, -O-$NO_2$ oder durch Phenyl, Phenoxy, Phenylthio oder Pyridyl substituiert ist, die ihrerseits durch Fluor, Chlor, Methyl, Methoxy, Methylthio, Trifluormethyl oder Trifluormethoxy substituiert sein können, oder

der Kohlenwasserstoffrest gegebenenfalls durch eine Gruppe der Formel -$CO_2R^{17}$, -NH-$SO_2$X und/oder -$NR^{20}R^{21}$ substituiert ist, worin

$R^{17}$ Wasserstoff, Methyl oder Ethyl bedeutet,

$R^{20}$ und $R^{21}$ gleich oder verschieden sind und Wasserstoff oder einen geradkettigen, verzweigten oder cyclischen, gesättigten oder ungesättigten Kohlenwasserstoffrest mit bis zu 5 Kohlenstoffatomen bedeuten, der gegebenenfalls durch Phenyl substituiert ist, das seinerseits durch Fluor, Chlor, Methyl oder Methoxy substituiert sein kann, oder

$R^{20}$ und $R^{21}$ gemeinsam unter Einbezug des Stickstoffatoms einen Piperidin- oder Piperazinring bilden, der gegebenenfalls durch Methyl, Ethyl, Phenyl oder Benzyl substituiert sein kann,

X Phenyl bedeutet, das gegebenenfalls durch Methyl substituiert ist,

$R^3$ für Cyano oder Nitro steht,

oder

$R^3$ und $R^4$ gemeinsam einen Rest der Formel

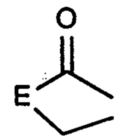

bilden,
worin

E ein Sauerstoff- oder Schwefelatom oder die $-CH_2$-Gruppe bedeutet,

$R^5$ für einen cyclischen, geradkettigen oder verzweigten, gesättigten oder ungesättigten Kohlenwasserstoffrest mit bis zu 6 Kohlenstoffatomen steht, der gegebenenfalls durch Sauerstoff oder Schwefel unterbrochen ist,
und der gegebenenfalls durch Cyclopropyl, Cyclobutyl, Cyclopentyl, Cyclohexyl, Fluor, Chlor, Cyano, Hydroxy oder durch Phenyl, Phenyloxy, Phenylthio oder durch einen 5- bis 7-gliedrigen, gesättigten oder ungesättigten Heterocyclus mit bis zu 2 Heteroatomen aus der Reihe S, N oder O substituiert ist, wobei die Cyclen ihrerseits durch Fluor, Chlor, Methyl, Methoxy, Trifluormethyl oder Trifluormethoxy oder durch eine Gruppe der Formel $-NR^{24}R^{25}$ substituiert sein können,
worin

$R^{24}$ und $R^{25}$ Wasserstoff, Phenyl, Benzyl oder geradkettiges oder verzweigtes Alkyl mit bis zu 4 Kohlenstoffatomen bedeuten,
oder der Kohlenwasserstoffrest gegebenenfalls durch eine Gruppe der Formel $-NR^{29}R^{30}$ substituiert ist,
worin

$R^{29}$ und $R^{30}$ gleich oder verschieden sind und Wasserstoff, Phenyl oder geradkettiges oder verzweigtes Alkyl mit bis zu 4 Kohlenstoffatomen bedeuten, das gegebenenfalls durch Phenyl substituiert ist,
oder

$R^5$ für einen 5- bis 6-gliedrigen, gesättigten oder ungesättigten Heterocyclus mit bis zu 2 Heteroatomen aus der Reihe S, N oder O steht, der gegebenenfalls durch Fluor, Chlor, Methyl, Methoxy, Methylthio oder Trifluormethyl substituiert ist,
oder

$R^5$ für eine Gruppe der Formel $D-R^{35}$ steht,
worin

D ein Sauerstoff- oder Schwefelatom bedeutet,
und

$R^{35}$ Phenyl bedeutet, das gegebenenfalls durch Fluor, Chlor, Methyl, Methoxy, Methylthio, Trifluormethyl, Amino oder durch $C_1$-$C_2$-Mono- oder Dialkylamino substituiert ist,
oder

$R^{35}$ Wasserstoff, einen cyclischen, geradkettigen oder verzweigten, gesättigten oder ungesättigten Kohlenwasserstoffrest mit bis zu 6 Kohlenstoffatomen bedeutet, der gegebenenfalls durch Fluor, Chlor oder Phenyl substituiert ist,

und deren Salze,
mit der Maßgabe, daß $R^5$ nicht für gegebenenfalls halogensubstituiertes Pyridyl oder Thienyl stehen darf.

**5.** Verfahren zur Herstellung von Verbindungen der allgemeinen Formel (I)

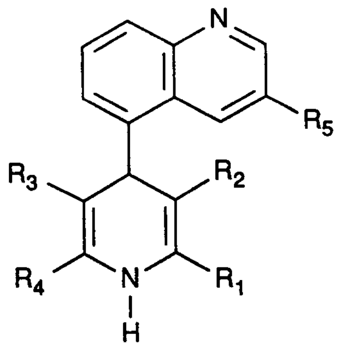

(I)

in welcher

$R^1$ und $R^4$ gleich oder verschieden sind und für Wasserstoff, Amino, Cyano, Formyl, Trifluormethyl oder für geradkettiges oder verzweigtes Alkyl mit bis zu 8 Kohlenstoffatomen stehen, das gegebenenfalls durch Hydroxy oder durch eine Gruppe der Formel - $NR^6R^7$, -O-CO-$R^8$, -O-$(CH_2)_a$-$OR^{8'}$ oder -O-$(CH_2)_{b'}$-$NR^9R^{10}$ substituiert ist, worin

$R^6$, $R^7$, $R^9$ und $R^{10}$ gleich oder verschieden sind und Wasserstoff, Phenyl oder geradkettiges oder verzweigtes Alkyl mit bis zu 6 Kohlenstoffatomen bedeuten,

$R^8$ und $R^{8'}$ gleich oder verschieden sind und geradkettiges oder verzweigtes Alkyl mit bis zu 6 Kohlenstoffatomen bedeuten, und

a und b gleich oder verschieden sind und eine Zahl 2, 3, 4 oder 5 bedeuten,

$R^2$ für eine Gruppe der Formel - CO-$NR^{11}R^{12}$ oder -CO-A-$R^{13}$ steht, worin

$R^{11}$ und $R^{12}$ gleich oder verschieden sind und Wasserstoff, einen geradkettigen, verzweigten oder cyclischen, gesättigten oder ungesättigten Kohlenwasserstoffrest mit bis zu 8 Kohlenstoffatomen bedeuten, der gegebenenfalls durch Halogen, Hydroxy, Cyano oder durch Aryl, Aryloxy, Arylthio mit jeweils 6 bis 10 Kohlenstoffatomen oder durch einen 5-bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder

Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder

$R^{11}$ und $R^{12}$ gemeinsam unter Einbezug des Stickstoffatoms einen 3-bis 8-gliedrigen, gesättigen oder ungesättigten Heterocyclus bilden, der gegebenenfalls durch ein Sauerstoffatom oder durch einen Rest der Formel $S(O)_d$, - CO- oder -$NR^{15}$ unterbrochen sein kann, worin

d eine Zahl 0, 1 oder 2 bedeutet,

$R^{15}$ Wasserstoff oder Aryl mit 6 bis 10 Kohlenstoffatomen bedeutet, das gegebenenfalls bis zu 2-fach gleich oder verschieden durch

Halogen, Cyano oder durch geradkettiges oder verzweigtes Alkyl, Alkoxy, Alkylthio, Alkoxycarbonyl mit jeweils bis zu 8 Kohlenstoffatomen, Halogenalkyl, Halogenalkoxy oder Halogenalkylthio mit jeweils bis zu 4 Kohlenstoffatomen substituiert ist, oder

einen cyclischen, gesättigten oder ungesättigten, geradkettigen oder verzweigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkoxy oder Alkylthio mit jeweils bis zu 4 Kohlenstoffatomen, Halogen, Aryl mit 6 bis 10 Kohlenstoffatomen, einen 5-bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das seinerseits durch Aryl mit 6 bis 10 Kohlenstoffatomen substituiert sein kann,

A eine direkte Bindung oder ein Sauerstoffatom bedeutet,

$R^{13}$ Wasserstoff oder Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeutet, wobei die Cyclen gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder

einen cyclischen, geradkettigen oder verzweigten, gesättigten oder ungesättigten Kohlenwasserstoffrest mit bis zu 10 Kohlenstoffatomen bedeutet, der gegebenenfalls bis zu 3-fach gleich oder verschieden durch Sauerstoff oder durch - CO-, -CO-NH-, -O-CO-, -CO-O-, -NH-CO-, $-SO_2-NH-$, $-NH-SO_2-$, $-S(O)_e-$oder $-NR^{16}-$ unterbrochen ist,

worin

e die oben angegebene Bedeutung von d hat und mit dieser gleich oder verschieden ist,

$R^{16}$ die oben angegebene Bedeutung von $R^{15}$ hat und mit dieser gleich oder verschieden ist,

oder der Kohlenwasserstoffrest gegebenenfalls bis zu 3-fach gleich oder verschieden durch Aryliden mit 6 bis 10 Kohlenstoffatomen oder heterocyclische Reste der Formeln

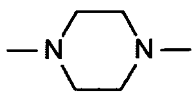 , 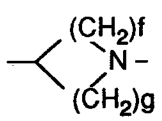 , 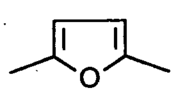 oder 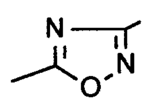

unterbrochen ist,

worin

f und g gleich oder verschieden sind und eine Zahl 1 oder 2 bedeuten,

und wobei Aryliden durch Halogen, Cyano oder durch geradkettiges oder verzweigtes Alkyl, Alkoxy, Alkylthio, Alkoxycarbonyl,

Halogenalkyl, Halogenalkoxy oder Halogenalkylthio mit jeweils bis zu 4 Kohlenstoffatomen substituiert sein kann,

und der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, - $O-NO_2$, geradkettiges oder verzweigtes Alkylthio, Alkoxy oder Acyloxy mit jeweils bis zu 8 Kohlenstoffatomen oder durch Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, wobei die Cyclen ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder

der Kohlenwasserstoffrest gegebenenfalls durch eine Gruppe der Formel $-CO_2-R^{17}$, $-CONR^{18}R^{19}$, - $NR^{20}R^{21}$ $-NH-SO_2-X$ und/oder $-NR^{22}-CO_2R^{23}$ substituiert ist,

worin

$R^{17}$ die oben angegebene Bedeutung von $R^{15}$ hat und mit dieser gleich oder verschieden ist

und

$R^{18}$, $R^{19}$, $R^{20}$, $R^{21}$, $R^{22}$ und $R^{23}$ die oben angegebene Bedeutung von $R^{11}$ und $R^{12}$ haben und mit diesen gleich oder verschieden sind,

X Phenyl bedeutet, das gegebenenfalls durch Methyl substituiert ist,

$R^3$ für Cyano, Nitro oder Formyl steht,

oder

$R^3$ und $R^4$ gemeinsam einen Rest der Formel

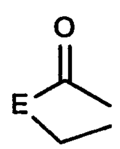

bilden,

worin

E ein Sauerstoff-oder Schwefelatom oder die - $CH_2$-Gruppe bedeutet,

$R^5$ für einen cyclischen, geradkettigen oder verzweigten, gesättigten oder ungesättigten Kohlenwasserstoffrest mit bis zu 12 Kohlenstoffatomen steht, der gegebenenfalls bis zu 2-fach gleich oder verschieden durch Sauerstoff oder Schwefel unterbrochen ist,

und der gegebenenfalls bis zu 3-fach gleich oder verschieden durch Cycloalkyl mit 3 bis 8 Kohlenstoffatomen, geradkettiges oder verzweigtes Acyloxy mit bis zu 4 Kohlenstoffatomen, Halogen, Nitro, Cyano, Hydroxy oder durch Aryl, Aryloxy oder Arylthio mit jeweils 6 bis 10 Kohlenstoffatomen oder durch einen 5-bis 7-gliedrigen, gesättigten oder ungesättigen gegebenenfalls kondensierten Heterocyclus mit bis zu 5 Heteroatomen aus der Reihe S, N oder O substituiert ist, wobei die Cyclen ihrerseits bis zu 3-fach gleich oder verschieden durch Halogen, Cyano, Nitro, Hydroxy oder durch geradkettiges oder verzweigtes Alkyl oder Alkoxy mit jeweils bis zu 4 Kohlenstoffatomen, Trifluormethyl, Trifluormethoxy,

Trifluormethylthio oder durch eine Gruppe der Formel -$NR^{24}R^{25}$ substituiert sein können,
worin

$R^{24}$ und $R^{25}$ die oben angegebene Bedeutung von $R^{11}$ und $R^{12}$ haben und mit dieser gleich oder verschieden sind,

oder der Kohlenwasserstoffrest gegebenenfalls durch eine Gruppe der Formel - $CO_2$-$R^{26}$, - $CONR^{27}R^{28}$, - $NR^{29}R^{30}$, - $NR^{31}$-$CO_2R^{32}$ oder -$NR^{33}$-$SO_2R^{34}$ substituiert ist,
worin

$R^{26}$ die oben angegebene Bedeutung von $R^{15}$ hat und mit dieser gleich oder verschieden ist
und

$R^{27}$, $R^{28}$, $R^{29}$, $R^{30}$, $R^{31}$, $R^{32}$, $R^{33}$ und $R^{34}$ die oben angegebene Bedeutung von $R^{11}$ und $R^{12}$ haben und mit diesen gleich oder verschieden sind,
oder

$R^5$ für einen 5-bis 7-gliedrigen, gesättigten oder ungesättigten Heterocyclus mit bis zu 4 Heteroatomen aus der Reihe S, N oder O steht, der gegebenenfalls bis zu 3-fach gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono-oder - Dialkylamino substituiert ist,
oder

$R^5$ für eine Gruppe der Formel D-$R^{35}$ steht,
worin

D die CO-oder - $S(O)_h$-Gruppe oder ein Sauerstoffatom bedeutet,
worin

h eine Zahl 0, 1 oder 2 bedeutet,
und

$R^{35}$ Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeutet, die gegebenenfalls bis zu 3-fach gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono-oder -Dialkylamino substituiert sind,
oder

$R^{35}$ Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel unterbrochen ist,

und der gegebenenfalls durch Halogen, Aryl, Aryloxy oder Arylthio mit jeweils 6 bis 10 Kohlenstoffatomen oder durch einen 5-bis 7-gliedrigen, gesättigten oder ungesättigen Heterocyclus mit bis zu 3 Heteroatomen aus der Reihe S, N oder O substituiert ist, wobei die Cyclen ihrerseits durch Halogen, Trifluormethyl, Methyl, Methoxy, Nitro oder Methylthio substituiert sein können,
oder

durch eine Gruppe der Formel - $NR^{36}R^{37}$ substituiert ist

worin

$R^{36}$ und $R^{37}$ die oben angegebene Bedeutung von $R^{11}$ und $R^{12}$ haben und mit dieser gleich oder verschieden sind,

und deren Salze,
mit der Maßgabe, daß $R^5$ nicht für gegebenenfalls halogensubstituiertes Pyridyl oder Thienyl stehen darf,
dadurch gekennzeichnet, daß man im Fall, daß
$R^3$ für Cyano, Nitro oder Formyl steht,
[A] Verbindungen der allgemeinen Formel (II)

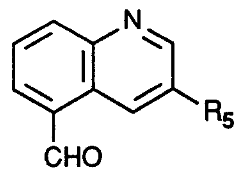

(II)

in welcher
$R^5$ die oben angegebene Bedeutung hat,
zunächst mit Acylverbindungen der allgemeinen Formel (III)

$R^3$-CO-$CH_2$-$R^4$ (III)

in welcher
$R^3$ und $R^4$ die oben angegebene Bedeutung haben
gegebenenfalls unter Isolierung der Ylidenverbindungen der allgemeinen Formel(IV)

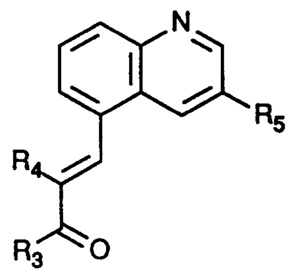

(IV)

in welcher
$R^3$, $R^4$ und $R^5$ die oben angegebene Bedeutung haben
umsetzt, anschließend mit Verbindungen der Formel (V)

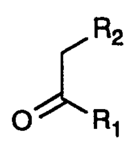

(V)

in welcher
$R^1$ und $R^2$ die oben angegebene Bedeutung haben,
und einer reaktiven Ammoniumverbindung, oder direkt mit Enamino-Verbindungen der allgemeinen Formel (VI)

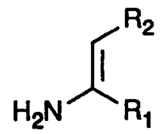

(VI)

in welcher
$R^1$ und $R^2$ die oben angegebene Bedeutung haben,
in inerten Lösemitteln umsetzt,
und
[B] Verbindungen der allgemeinen Formel (II) zunächst mit Verbindungen der allgemeinen Formel (V), gegebenenfalls unter Isolierung der Ylidenverbindungen der allgemeinen Formel (VII)

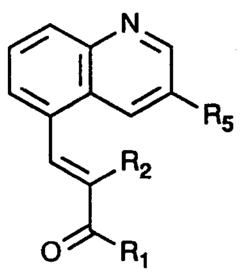

(VII)

in welcher
$R^1$, $R^2$ und $R^5$ die oben angegebene Bedeutung haben,
umsetzt und anschließend entweder mit Verbindungen der allgemeinen Formel (III) in Gegenwart von Ammoniumverbindungen oder direkt mit Verbindungen der allgemeinen Formel (VIII)

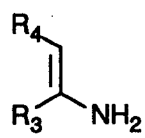

(VIII)

in welcher
$R^3$ und $R^4$ die oben angegebenene Bedeutung haben,
umsetzt
oder
[C] im Fall, daß $R^3$ und $R^4$ zusammen einen Rest der Formel

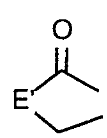

bilden,
worin
E' für ein Sauerstoff- oder Schwefelatom steht,
zunächst nach dem unter [A] und [B] aufgeführten Methoden Verbindungen der allgemeinen Formel (IX)

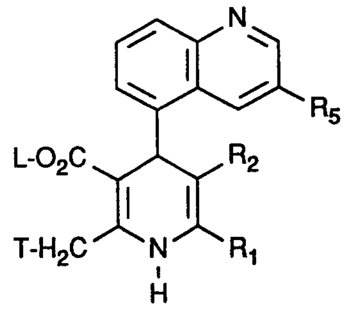

(IX)

in welcher
$R^1$, $R^2$ und $R^5$ die oben angegebene Bedeutung haben,
L für $C_1$-$C_4$-Alkyl steht
und
T für $C_1$-$C_4$-Acyloxy oder Acylthio steht,
herstellt und anschließend einen basischen oder sauren Ringschluß nach bekannten Methoden durchführt,
oder
[D] daß man im Fall, daß E für die -$CH_2$-Gruppe steht,
Verbindungen der allgemeinen Formel (II),zunächst mit Acylverbindungen der allgemeinen Formel (X)

L-CO-$CH_2$-$R^2$ (X)

in welcher
$R^2$ die oben angegebene Bedeutung hat
und
L die oben angegebene Bedeutung von $R^1$ hat, wobei im Fall der Hydroxy- und/oder Aminofunktionen, diese gegebenenfalls in geschützter Form vorliegen,
gegebenenfalls unter Isolierung der Ylidenverbindungen der allgemeinen Formel (XI)

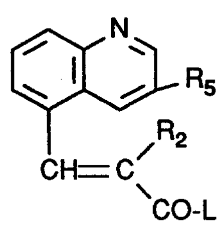

(XI)

in welcher
$R^2$, $R^5$ und L die oben angegebene Bedeutung haben
umsetzt, anschließend mit der Verbindung der Formel (XII)

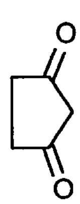

(XII)

und einer reaktiven Ammoniumverbindung, gegebenenfalls unter Isolierung der Zwischenprodukte der allgemeinen Formel (XIII)

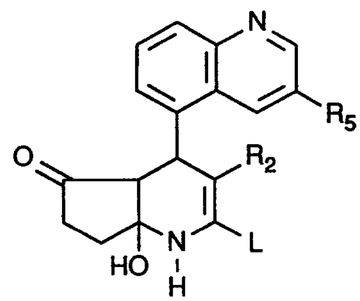

(XIII)

in welcher
$R^2$, $R^3$ und L die oben angegebene Bedeutung haben,
in inerten Lösemitteln umsetzt,
und dann in einem letzten Schritt, gegebenenfalls in Anwesenheit eines Hilfsmittels, Wasser abscheidet.

**6.** Verbindungen der allgmeinen Formel (I) gemaß Anspruch 1 zur Verwendung bei der Behandlung von Herz-Kreislauferkrankungen.

**7.** Arzneimittel enthaltend mindestens eine Verbindung der allgemeinen Formel (I) gemäß Anspruch 1.

**8.** Verfahren zur Herstellung von Arzneimitteln, dadurch gekennzeichnet, daß man mindestens eine Verbindung der allgemeinen Formel (I) gemaß Anspruch 1 gegebenenfalls mit üblichen Hilfs- und Trägerstoffen in eine geeignete Applikationsform überführt.

**9.** Verwendung von Verbindungen der allgemeinen Formel (I) gemäß Anspruch 1 in Arzneimitteln mit positiv ionotroper Wirkung.

**10.** Aldehyde der allgemeinen Formel (II)

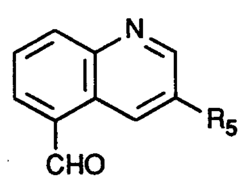

(II)

in welcher
$R^5$ für einen cyclischen, geradkettigen oder verzweigten, gesättigten oder ungesättigten Kohlenwasserstoffrest mit bis zu 12 Kohlenstoffatomen steht, der gegebenenfalls bis zu 2-fach gleich oder verschieden durch Sauerstoff oder Schwefel unterbrochen ist,
und der gegebenenfalls bis zu 3-fach gleich oder verschieden durch Cycloalkyl mit 3 bis 8 Kohlenstoffatomen, geradkettiges oder verzweigtes Acyloxy mit bis zu 4 Kohlenstoffatomen, Halogen, Nitro, Cyano, Hydroxy oder durch Aryl, Aryloxy oder Arylthio mit jeweils 6 bis 10 Kohlenstoffatomen oder durch einen 5-bis 7-gliedrigen, gesättigten oder ungesättigen gegebenenfalls kondensierten Heterocyclus mit bis zu 5 Heteroatomen aus der Reihe S, N oder O substituiert ist, wobei die Cyclen ihrerseits bis zu 3-fach gleich oder verschieden durch Halogen, Cyano, Nitro, Hydroxy oder durch geradkettiges oder verzweigtes Alkyl oder Alkoxy mit jeweils bis zu 4 Kohlenstoffatomen, Trifluormethyl, Trifluormethoxy, Trifluormethylthio oder durch eine Gruppe der Formel -$NR^{24}R^{25}$ substituiert sein können,
worin

$R^{24}$ und $R^{25}$ — die oben angegebene Bedeutung von $R^{11}$ und $R^{12}$ haben und mit dieser gleich oder verschieden sind,
wobei

$R^{11}$ und $R^{12}$ — gemeinsam unter Einbezug des Stickstoffatoms einen 3- bis 8-gliedrigen, gesättigen oder ungesättigten Heterocyclus bilden, der gegebenenfalls durch ein Sauerstoffatom oder durch einen Rest der Formel $S(O)_d$, - CO- oder - $NR^{15}$ unterbrochen sein kann,
worin

d — eine Zahl 0, 1 oder 2 bedeutet,

$R^{15}$ — Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl mit jeweils bis zu 8 Kohlenstoffatomen, Halogenalkyl, Halogenalkoxy oder Halogenalkylthio mit jeweils bis zu 4 Kohlenstoffatomen substituiert ist, oder
einen cyclischen, gesättigten oder ungesättigten, geradkettigen oder verzweigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkoxy oder Alkylthio mit jeweils bis zu 4 Kohlenstoffatomen, Halogen, Aryl mit 6 bis 10 Kohlenstoffatomen, einen 5-bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das seinerseits durch Aryl mit 6 bis 10 Kohlenstoffatomen substituiert sein kann,

oder der Kohlenwasserstoffrest gegebenenfalls durch eine Gruppe der Formel - $CO_2$-$R^{26}$, - $CONR^{27}R^{28}$, - $NR^{29}R^{30}$, - $NR^{31}$-$CO_2R^{32}$ oder -$NR^{33}$-$SO_2R^{34}$ substituiert ist,
worin

$R^{26}$ — die oben angegebene Bedeutung von $R^{15}$ hat und mit dieser gleich oder verschieden ist
und

$R^{27}$, $R^{28}$, $R^{29}$, $R^{30}$, $R^{31}$, $R^{32}$, $R^{33}$ und $R^{34}$ — die oben angegebene Bedeutung von $R^{11}$ und $R^{12}$ haben und mit diesen gleich oder verschieden sind,
oder

$R^5$ — für einen 5-bis 7-gliedrigen, gesättigten oder ungesättigten Heterocyclus mit bis zu 4 Heteroatomen aus der Reihe S, N oder O steht, der gegebenenfalls bis zu 3-

| | |
|---|---|
| | fach gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono-oder - Dialkylamino substituiert ist, |
| | oder |
| $R^5$ | für eine Gruppe der Formel D-$R^{35}$ steht, |
| | worin |
| D | die CO-oder - $S(O)_h$-Gruppe oder ein Sauerstoffatom bedeutet, |
| | worin |
| h | eine Zahl 0, 1 oder 2 bedeutet, |
| | und |
| $R^{35}$ | Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeutet, die gegebenenfalls bis zu 3-fach gleich oder verschieden durch Halogen, Amino, Cyano, Nitro oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen oder durch $C_1$-$C_4$-Mono-oder-Dialkylamino substituiert sind, |
| | oder |
| $R^{35}$ | Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder Schwefel unterbrochen ist, und der gegebenenfalls durch Halogen, Aryl, Aryloxy oder Arylthio mit jeweils 6 bis 10 Kohlenstoffatomen oder durch einen 5-bis 7-gliedrigen, gesättigten oder ungesättigen Heterocyclus mit bis zu 3 Heteroatomen aus der Reihe S, N oder O substituiert ist, wobei die Cyclen ihrerseits durch Halogen, Trifluormethyl, Methyl, Methoxy, Nitro oder Methylthio substituiert sein können, oder |
| | durch eine Gruppe der Formel - $NR^{36}R^{37}$ substituiert ist |
| | worin |
| $R^{36}$ und $R^{37}$ | die oben angegebene Bedeutung von $R^{11}$ und $R^{12}$ haben und mit dieser gleich oder verschieden sind, |

mit der Maßgabe, daß $R^5$ nicht für gegebenenfalls halogensubstituiertes Pyridyl oder Thienyl stehen darf.

**11.** Verfahren zur Herstellung von Verbindungen der allgemeinen Formel (II) gemäß Anspruch 10, dadurch gekennzeichnet, daß man
4-Amino-3-hydroxyphthalid der Formel (XIV)

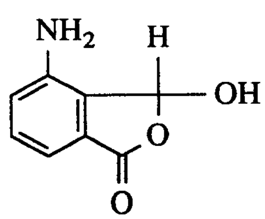

(XIV)

entweder nach Isolierung oder direkt in situ nach Hydrierung von 4-Nitro-3-hydroxyphthalid der Formel

(XV)

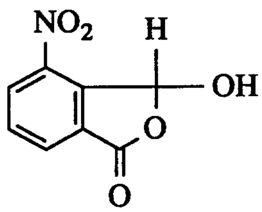

(XV)

mit Verbindungen der allgemeinen Formel (XVI)

$R^5$-$CH_2$-CHO (XVI)

zu Verbindungen der allgemeinen Formel (XVII) cyclisiert

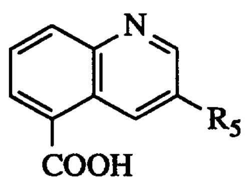

(XVII)

diese dann mit üblichen Reduktionsmitteln wie beispielsweise Lithiumaluminiumhydrid oder über ein gemischtes Anhydrid mit Natriumborhydrid in die Alkohole der allgemeinen Formel (XVIII)

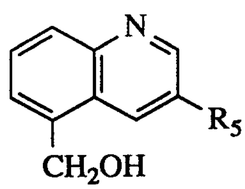

(XVIII)

überführt und diese dann entweder nach Isolierung oder direkt in situ mit Oxidationsmittel wie beispielsweise Mangandioxid zu Verbindungen der allgemeinen Formel (II) oxodiert,
wobei
$R^5$ die jeweils im Anspruch 10 angegebene Bedeutung hat.

Europäisches Patentamt

# EUROPÄISCHER RECHERCHENBERICHT

Nummer der Anmeldung
EP 94 10 5773

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| P,X | EP-A-0 538 690 (BAYER AG) 28. April 1993 | 1-11 | C07D401/04<br>A61K31/445<br>C07D211/90<br>C07D401/14<br>C07D215/14<br>C07D215/12 |
| X | EP-A-0 518 105 (BAYER AG) 16. Dezember 1992 | 1-11 | |
| X | EP-A-0 515 940 (BAYER AG) 2. Dezember 1992<br>siehe insbesondere die Beispiele | 1-11 | |
| X | EP-A-0 452 712 (BAYER AG) 23. Oktober 1991 | 1-11 | |
| Y | EP-A-0 451 654 (BAYER AG) 16. Oktober 1991 | 1-11 | |
| X,Y | EP-A-0 071 819 (BAYER AG) 16. Februar 1983 | 1-11 | |
| X | COLLECT.CZECH.CHEM.COMMUN.<br>Bd. 57 , 1992<br>Seiten 169 - 178<br>BARALDI,PG. ET AL. 'Synthesis and Calcium Antagonist Acitivity of Dialkyl 1,4-Dihydro-2,6-dimethyl-4(nitrogenous heteroaryl)-3,5-pyridine dicarboxylates'<br>siehe Seite 173,Scheme 3, Seite 177,erstes Beispiel | 10,11 | RECHERCHIERTE SACHGEBIETE (Int.Cl.5)<br>C07D<br>A61K |

Der vorliegende Recherchenbericht wurde für alle Patentansprüche erstellt

| Recherchenort | Abschlußdatum der Recherche | Prüfer |
|---|---|---|
| MÜNCHEN | 19. September 1994 | Stellmach, J |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)